(19) Europäisches Patentamt
European Patent Office
Office européen des brevets

(11) **EP 1 782 787 A1**

(12) **EUROPEAN PATENT APPLICATION**
published in accordance with Art. 158(3) EPC

(43) Date of publication:
**09.05.2007 Bulletin 2007/19**

(51) Int Cl.:
*A61H 39/06* (2006.01)    *A61F 7/08* (2006.01)

(21) Application number: **05765770.2**

(22) Date of filing: **14.07.2005**

(86) International application number:
**PCT/JP2005/013006**

(87) International publication number:
**WO 2006/006653 (19.01.2006 Gazette 2006/03)**

(84) Designated Contracting States:
**DE FR GB**

(30) Priority: **14.07.2004 JP 2004207834**

(71) Applicant: **Mycoal Products Corporation**
**Tochigi-chi, Tochigi 328-0067 (JP)**

(72) Inventor: **DODO, Toshihiro,**
**MYCOAL PRODUCTS CORPORATION,**
**Tochigi-shi, Tochigi 3280067 (JP)**

(74) Representative: **Thun, Clemens**
**Mitscherlich & Partner**
**Sonnenstrasse 33**
**80331 München (DE)**

(54) **MICRO-HEATER AND METHOD FOR MANUFACTURE THEREOF**

(57)    To provide a microheater which gives a desired temperature and starts an exothermic reaction upon immediate contact with air and in which plural microheaters can be chained or incorporated in a packaging material.

A microheater having a heat generating composition molded body made of a moldable heat generating composition containing surplus water as a connecting substance accommodated in an air-permeable accommodating bag, which is **characterized in that** the accommodating bag is made of a heat seal layer-containing substrate and a covering material and has an exothermic part as formed by laminating a heat generating composition molded body as molded on the substrate which is substantially planar and does not have a pocket, an accommodating division and an accommodating section, covering by the covering material and heat sealing the periphery of the heat generating composition molded body; that the moldable heat generating composition contains, as essential components, an iron powder, a carbon component, a reaction accelerator and water, has a content of water in the moldable heat generating composition of from 1 to 60 %, does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air; that a volume of the heat generating composition molded body is from 0.1 to 30 cm$^3$, and a ratio of a capacity of the exothermic part to the volume of the heat generating composition molded body is from 0.6 to 1.0; and that a maximum height of the exothermic part is from 0.1 to 10 mm.

*FIG.2*

EP 1 782 787 A1

**Description**

[Technical Field]

The present invention relates to a microheater containing a heat generating composition molded boy resulting from molding a moldable heat generating composition using surplus water as a connecting substance and containing, as essential components, an iron powder, a carbon component, a reaction accelerator and water.

[Background Art]

As a thermal stimulus in a minute region, moxibustion is known, and thermal moxibustion goods are proposed as the application of a minute microheater. That is, moxibustion in the Eastern medicine improves circulation of the blood by a thermal local stimulus to be carried out against a meridian point (acupuncture point) on the pathway as spread in the body, and is approved to be effective in the remedy or recovery of stiffness of the shoulders, neuralgia, lower-back pain, muscular fatigue, etc. Then, according to the moxibustion which has been generally carried out so far, moxa as prepared by drying leaves of a mugwort is directly stuck onto an affected part (acupuncture point) of the skin and burnt, thereby giving a thermal stimulus to the affected part. However, in this case, there are involved various problems in view of use such that the heat which the skin receives is strong and that the scar of a burn remains.
Furthermore, there has hitherto been provided an electric thermal moxibustion unit or the like. However, this is not only expensive in its device but also inconvenient such that an electric source is required, and therefore, it has not been spread yet. Then, there have been provided thermal moxibustion goods which are prepared by installing moxa as formed in a pillar form on a pedestal formed of thick paper, etc. and thermal moxibustion goods which are prepared by sealing a composition capable of causing heat generation by an oxidation reaction in a disk-like container. These have been spread.
The former is used by sticking the pedestal onto an affected part of the skin. Since the pedestal becomes a buffer of heat in burning the moxa, not only the action of the strong heat to the skin is relieved, but also what the scar of a burn remains is extremely scarce. Furthermore, the latter is used by sticking the container onto an affected part of the skin. The thermal moxibustion is carried out by causing heat generating by making oxygen in air act to an internal composition by boring the container or the like and simultaneously making this heat act to the affected part. In this case, since the temperature of the heat generation due to the oxidation does not become high, it can be used without causing a phenomenon in which the strong heat acts to the skin or the scar of a burn remains.
For example, Patent Document 1 proposes thermal moxibustion goods made of a drug and a microheater.
Also, Patent Document 2 proposes thermal moxibustion goods made of moxa and a microheater.
On the other hand, as a microheater having a size larger than those described above, a microheater using a heat generating composition utilizing an oxidation reaction of a metal such as iron in a form of a powder or granule, a viscous body, a creamy body, etc. is prepared. This microheater is very excellent in view of costs, safety, exothermic temperature, and the like and is already provided for practical use, for example, as a so-called chemical body warmer in which the microheater is filled in an air-permeable bag.
In addition, in order to obtain a more comfortable feeling for use, there have been proposed various heat generating compositions which design to have shape holding properties and to hold exothermic characteristics while using a thickener, a binding agent, etc. in quest of prevention of deviation of a heat generating composition and fitness to various kinds of shapes.
For example, Patent Document 3 proposes a process for producing a heat generating composition as granulated so as to have an average particle size of 0.5 mm or more and a process for producing a heat generating composition having an improved granular strength after granulation by blending from 10 to 20 parts by weight of an adhesive binder component such as water glass and polyvinyl alcohol with addition water.
Also, Patent Document 4 proposes a throwaway body warmer composed of a heat generating composition having shape holding characteristics by adding a powdered thickener such as corn starch and potato starch.
Also, Patent Document 5 proposes a throwaway body warmer using a heat generating composition which does not cause deviation of the contents by adding an excipient such as α-starch and carboxymethyl cellulose.
Also, Patent Document 6 proposes a solid heat generating composition as prepared by mixing a binding agent such as CMC in a powdered or granular heat generating composition and compression molding the mixture.
Also, Patent Document 7 proposes a microheater as prepared by using a crosslinking agent, etc. and a water absorptive polymer and integrating them under pressure.
Also, Patent Document 8 proposes a heat generating composition in an ink form and/or a creamy form containing a thickener such as carboxymethyl cellulose, a microheater and a process for producing the same.
Also, Patent Document 9 proposes a heat generating composition molded body using a binding agent, the surface of which is covered by an air-permeable film such as CMC, thereby designing to hold the shape.

Also, Patent Document 10 and Patent Document 11 propose that a heat generating composition containing a thickener such as carboxymethyl cellulose is processed into an ink form and/or a creamy form, water works as a barrier layer, and after removing a fixed amount of water by water absorption or other means, the heat generating composition causes heat generation, in which the shape is changed from a conventional rectangle to a foot shape or an elliptical shape so as to adapt to the outline of a body to be warmed.

Also, Patent Document 12 proposes a heat cell in which an accommodating pocket is previously prepared in a film layer substrate, a granular heat generating composition is filled in the pocket, the material is covered and sealed by another substrate, and water is then poured into a pinhole of the substrate, thereby preparing a microheater.

Also, Patent Document 13, Patent Document 14, Patent Document 15 and Patent Document 16 each proposes a microheater in which a heat generating composition exothermic part is sectioned into plural divisions by using a heat generating composition using a flocculant aid such as gelatin and corn syrup or a dry binding agent such as carboxymethyl cellulose for the purpose of increasing fitness to the body, etc., or by using a granular heat generating composition and a substrate having an accommodating pocket.

However, in the conventional thermal moxibustion goods, there was some possibility that the warmth does not sufficiently act to the affected part, and there was also a problem that a thermal moxibustion effect becomes insufficient. Furthermore, according to thermal moxibustion goods using a microheater, in a production system of a microheater of a filling system for filling a powdered heat generating composition while sealing a packaging material as in the conventional system, the size is limited. Thus, in the mass production of a small microheater for moxibustion, there were problems in the size, the volume of manufacture, the yield, and the like. Also, the quality and costs of thermal moxibustion goods were problematic.

Furthermore, in a large-sized microheater of the size of conventional throwaway body warmers, following spreading in utilization of throwaway body warmers which are aimed to be applied to various places of a human body such shoulders, arms, a neck and feet, even if a heat generating composition is hardened by a thickener, etc., there were encountered problems that in a single packed state, for example, bonding retention is difficult so that the dropping easily occurs and that a strong uncomfortable feeling is caused in wearing. Such problems are promoted due to a lowering of flexibility as caused by blocking following progression of a reaction of the microheater. There was also encountered a problem that a stretched film which forms an accommodating bag is shrunk and curled due to heat generation so that an end part of a single packaging bag rides up, whereby a body warmer as bonded and held easily peels away and drops due to catch therein.

Furthermore, so far, a microheater was produced by a filling system or produced by filling a heat generating composition containing a flocculant and a binding agent in a packaging material having accommodating divisions resulting from molding in vacuo an agglomerate or compressed body. Moreover, a microheater was produced by previously preparing a filling pocket in a substrate, filling a granular heat generating composition in the pocket and covering a packaging material thereon, followed by sealing.

Furthermore, in the case of producing a microheater having sectioned exothermic parts by using a powdered heat generating composition or a granular heat generating composition as a heat generating composition, according to a method using a filling system, since the powdered heat generating composition or granular heat generating composition is accommodated in an accommodating body in a partially sealed bag form and the whole is then sealed, there was a limit in size of a sectional region in view of the production. That is, according to a method for filling a powdered heat generating composition or a granular heat generating composition while partially sealing, it was mechanically substantially impossible to produce a microheater having a plural number of small-sized sectional regions, and additionally, there was caused a problem due to shortage in sealing as caused by incorporation of the heat generating composition into a seal part or the like. In particular, it was substantially impossible to continuously produce one having a partial shape having a size of not more than 20 mm or one having a small shape of not more than 20 mm. Furthermore, according to a method using a rotary magnet system, in a method using four rotary structures, a complicated operation must be carried out such that a concave having a magnet is provided on the circumferential surface of each of the three rotary structures A, C and D in the bottom thereof; a magnet is provided on the planar circumferential surface of the other rotary structure B in the bottom thereof; the rotary structure A and the rotary structure B are rotated adjacently in the opposite direction to each other; the rotary structure B and the rotary structure C are rotated adjacently in the opposite direction to each other; the rotary structure C and the rotary structure D are rotated adjacently in the opposite direction to each other; and the magnets are rotated while synchronizing the concave of the rotary structure A with the magnet of the rotary structure B in a facing state, synchronizing the magnet of the rotary structure B with the concave of the rotary structure C in a facing state, and synchronizing the magnet of the rotary structure C with the concave of the rotary structure D, and that the structure is complicated. Accordingly, there were encountered problems that the operation at the time of forming an exothermic layer is troublesome and that a device to be used is complicated and expensive, is liable to cause a fault, takes a long time to do the maintenance and is inconvenient for handling.

Furthermore, according to a method using a pocket system, a heat generating composition containing a flocculant and a binding agent is used and a dry powdered mixture of an exothermic component containing a flocculant and a binding agent is filled in a concave pocket as previously prepared in a packaging material directly or after compressing it to form a granule, a pellet, a tablet or a scrub, followed by optionally carrying out compression to prepare an exothermic part. Furthermore, in comparison with a microheater in which a flocculant and a binding agent are not incorporated, one in a form in which a heat generating composition is hardened by a flocculant aid, etc. is deteriorated in exothermic performance, resulting in a problem in view of practical use.

[Patent Document 1] JP-A-7-136233
[Patent Document 2] JP-A-2000-254205
[Patent Document 3] JP-A-4-293989
[Patent Document 4] JP-A-6-343658
[Patent Document 5] JP-A-7-194641
[Patent Document 6] JP-A-59-189183
[Patent Document 7] WO 00/13626
[Patent Document 8] JP-A-9-75388
[Patent Document 9] JP-A-60-101448
[Patent Document 10] JP-A-9-276317
[Patent Document 11] JP-A-11-299817
[Patent Document 12] JP-T-11-508314
[Patent Document 13] JP-UM-A-6-26829
[Patent Document 14] JP-A-2000-288008
[Patent Document 15] JP-T-11-508786
[Patent Document 16] JP-T-2002-514104

[Disclosure of the Invention]

[Problems that the Invention is to Solve]

Accordingly, an object of the invention is to provide a microheater which gives an adjusted and held temperature and starts an exothermic reaction upon immediate contact with air and in which plural microheaters can be chained or incorporated in a packaging material. That is, an object of the invention is to provide a microheater in which thermal moxibustion can be achieved by making sufficient warmth act to the skin, a time required for the warmth to start to act can be shortened, and a high thermal moxibustion effect can be obtained by making a time when warmth or a warmth feeling acts long and holding it over a long period of time. In addition, another object of the invention is to provide a microheater which can be chained as plural sectioned exothermic sources, from which uniform, convenient and comfortable heat can be provided. Also, a still another object of the invention is to provide a microheater for providing uniform, convenient and comfortable heat, in which plural microheaters can be easily incorporated in a throwaway packaging material to be employed in wide and various body shapes.

[Means for Solving the Problems]

Then, in order to solve these conventional problems, the present inventors made extensive and intensive investigations, carried out various systematic experiments and achieved studies for the purpose of obtaining a microheater which gives an adjusted and held temperature and immediately reaches a detection temperature. As a result, they have attained the invention.
Specifically, as set forth in claim 1, a microheater of the invention is a microheater having a heat generating composition molded body made of a moldable heat generating composition containing surplus water as a connecting substance accommodated in an air-permeable accommodating bag, which is characterized in that:

1) the accommodating bag is made of a heat seal layer-containing substrate and a covering material and has an exothermic part as formed by laminating a heat generating composition molded body as molded on the substrate which is substantially planar and does not have a pocket, an accommodating division and an accommodating section, covering by the covering material and heat sealing the periphery of the heat generating composition molded body,
2) the moldable heat generating composition contains, as essential components, an iron powder, a carbon component, a reaction accelerator and water, has a content of water in the moldable heat generating composition of from 1 to 60 %, does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air,
3) a volume of the heat generating composition molded body is from 0.1 to 30 $cm^3$, and a ratio of the capacity of the exothermic part to the volume of the heat generating composition molded body a is from 0.6 to 1.0, and
4) a maximum height of the exothermic part is from 0.1 to 10 mm.

Also, a microheater as set forth in claim 2 is characterized in that in the microheater as set forth in claim 1, the shape of the heat generating composition molded body, the exothermic part and the microheater is at least one shape selected from the group consisting of a circular shape, a triangular shape, a star shape, a rectangular shape, a square shape, a flower shape, an elliptical shape, a cubic shape, a parallelepiped shape, a polygonal pyramidal shape, a conical shape, a pillar shape, an elliptic cylindrical shape, semi-pillar shape, a semi-elliptic cylindrical shape, a cylindrical shape, and a spherical shape.

Also, a microheater as set forth in claim 3 is characterized in that in the microheater as set forth in claim 2, the shape of the exothermic part is a pillar shape and has a diameter of from 1 to 50 mm and a maximum height of from 0.1 to 10 mm.

Also, a microheater as set forth in claim 4 is characterized in that in the microheater as set forth in claim 2, the shape of the exothermic part is a parallelepiped shape and has a maximum length of from 5 to 200 mm, a maximum width of from 1 to 50 mm and a maximum height of from 0.1 to 10 mm, and the exothermic part is formed by heat sealing the periphery of the heat generating composition molded body.

Also, a microheater as set forth in claim 5 is characterized in that in the microheater as set forth in claim 2, the shape of the exothermic part is an elliptic cylindrical shape and has a maximum width of from 3 to 30 mm.

Also, a microheater as set forth in claim 6 is characterized in that in the microheater as set forth in claim 2, the exothermic part has a maximum width of from 1 to 50 mm, a maximum height of from 0.1 to 10 mm and a longest length of from 5 to 200 mm.

Also, a microheater as set forth in claim 7 is characterized in that in the microheater as set forth in claim 2, the shape of the exothermic part is a cubic shape and has a maximum width of from 5 to 30 mm, and the exothermic part is formed by heat sealing the periphery of the heat generating composition molded body.

Also, a microheater as set forth in claim 8 is characterized in that in the microheater as set forth in claim 1, at least the heat generating composition molded body is compressed.

Also, a microheater as set forth in claim 9 is characterized in that in the microheater as set forth in claim 1, the heat seal part is formed by heat sealing after temporary adhesion by an adhesive layer as formed on the heat seal layer, and an adhesive component which constitutes the adhesive layer and a heat seal material component which constitutes the heat seal layer are copresent in the heat seal part.

Also, a microheater as set forth in claim 10 is characterized in that in the microheater as set forth in claim 1, the moldable heat generating composition contains a component resulting from a contact treatment of a mixture containing at least an iron powder, a carbon component, a reaction accelerator and water as essential components with an oxidizing gas.

Also, a microheater as set forth in claim 11 is characterized in that in the microheater as set forth in claim 1, the iron powder comprising particles, a surface of each of which is at least partially covered with an iron oxide film, the oxide film has a thickness of 3 nm or more, and the iron powder at least contains from 20 to 100 % by weight of an active iron powder particle having a region of an oxygen-free iron component in at least one region selected from a central part region of the iron powder particle and a region beneath the iron oxide film.

Also, a microheater as set forth in claim 12 is characterized in that in the microheater as set forth in claim 1, the iron powder comprising particles, a surface of each of which is at least partially covered with a wustite film and contains from 20 to 100 % by weight of an active iron powder having an amount of wustite of from 2 to 50 % by weight in terms of an X-ray peak intensity ratio to iron.

Also, a microheater as set forth in claim 13 is characterized in that in the microheater as set forth in claim 1, the moldable heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

Also, a microheater as set forth in claim 14 is characterized in that in the microheater as set forth in claim 1, 80 % or more of a non-water soluble solid component which constitutes the moldable heat generating composition has a particle size of not more than 300 $\mu$m and a maximum particle size of not more than 1 mm.

Also, a microheater as set forth in claim 15 is characterized in that in the microheater as set forth in claim 1, in the substrate or the covering material, a sticky layer is laminated as a fixing measure on at least a part of the exposed surface thereof.

As set forth in claim 16, a process for producing a microheater of the invention is a process for producing a microheater having a heat generating composition molded body accommodated in an air-permeable accommodating bag, which is characterized in that:

1) a moldable heat generating composition containing surplus water as a connecting substance is molded, the heat generating composition molded body is laminated on a substrate which is substantially planar and does not have an accommodating pocket, the heat generating composition molded body is covered by a covering material, and the periphery of the heat generating composition molded body is heat sealed to form an exothermic part,

2) the moldable heat generating composition contains, as essential components, an iron powder, a carbon component, a reaction accelerator and water, has a content of water in the moldable heat generating composition of from 1 to 60 %, does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air,

3) a volume of the heat generating composition molded body is from 0.1 to 30 cm$^3$, and a ratio of the capacity of the exothermic part to the volume of the heat generating composition molded body is from 0.6 to 1.0, and

4) a maximum height of the exothermic part is from 0.1 to 10 mm.

Also, a process for producing a microheater as set forth in claim 17 is characterized in that in the process for production a microheater as set forth in claim 16, at least the periphery of the heat generating composition molded body is heat sealed after temporary adhesion of the substrate and the covering material via a sticky layer.

Also, in the microheater, it is preferable that the adhesive layer is a non-hydrophilic adhesive layer, and an adhesive which constitutes the adhesive layer is a non-aromatic hot melt based adhesive.

Also, in the microheater, it is preferable that adhesive layer is a hydrophilic adhesive layer, and an adhesive which constitutes the adhesive layer contains, as essential components, a crosslinking type water absorptive polymer, a water-soluble polymer and a softener and has a content of the crosslinking type water absorptive polymer of from 3 to 80 % by weight.

Also, in the microheater, it is preferable that the adhesive layer does not contain a drug.

Also, in the microheater, it is preferable that the microheater reaches 40 °C or higher within 3 minutes after the start of heat generation and has a maximum temperature of 45 °C or higher.

Also, in the process for producing a microheater, it is preferable that the moldable heat generating composition is compressed within a die.

[Advantages of the Invention]

According to the invention, the following advantages are brought.

1) The microheater of the invention gives an adjusted and held temperature and immediately reaches a maximum temperature and is able to provide uniform, convenient and comfortable heat.

2) Since an adhesive layer-provided microheater is provided with a heat generating part for generating heat for thermal moxibustion and a warmth imparting layer to the skin, not only it is possible to impart a warm feeling to the skin within a short period of time after sticking the adhesive layer-provided microheater to the skin, but also it is possible to enhance a thermal stimulus to the skin and to obtain a high thermal moxibustion effect because of its good adhesion to the skin.

3) The adhesive layer-provided microheater of the invention is small in size and is able to enhance a thermal effect with good efficiency while concentrating a region. In the case of using a drug-containing sticky layer, it is possible to absorb the drug in the blood whose circulation has become active due to the thermal effect or the like, thereby more effectively circulating the drug into various parts of a living body. Thus, a local remedy effect is much more improved, a remedy effect of the whole body is much more improved, and a pharmacological effect is much more enhanced. As a result, the adhesive layer-provided microheater of the invention is extremely beneficial as medicinal goods.

4) Since the microheater of the invention is small in size, when it is used in symptoms accompanied with stiffness of a local part, pain, the cold, etc., for example, diseases including stiffness of shoulders, muscular pain, muscular stiffness, lower-back pain, the cold of arms and legs, neuralgia, rheumatism, bruise, and sprain, it reveals a remedy effect due to the warmth.

5) Since the microheater of the invention can be processed into a chained body in which plural microheaters are disposed at intervals, it can be applied to various outlines of the body over a wide range. Also, it is possible to give an adjusted and held temperature for the application of uniform, convenient and comfortable heat for the purpose of remedying a temporary or chronic pain.

6) In addition, since the microheater of the invention can be easily incorporated in a throwaway packaging material for fixing the body to be employed in wide and various body shapes, it can be applied to various outlines of the body over a wide range. Also, it is possible to give an adjusted and held temperature for the application of uniform, convenient and comfortable heat for the purpose of remedying a temporary or chronic pain.

7) In a thermal sticking agent made of the adhesive layer-provided microheater according to the invention, a substrate and a covering material, the both of which constitute a flat bag body, are formed of a non-stretchable material, and its production process is simple.

8) By using an iron powder having an oxygen-containing film of iron on the surface thereof, in the case of heat generating compositions having the same exothermic rising properties, the carbon component in the heat generating composition can be reduced. Thus, if the capacity is identical, a proportion of the iron powder increases so that the duration of the exothermic temperature can be more prolonged.

In the light of the above, the invention is concerned with a microheater using a heat generating composition molded body resulting from molding a moldable heat generating composition containing surplus water as a connecting substance, wherein the heat generating composition does not contain a flocculant aid, dry binding agent and flocculant but contains an appropriate amount of surplus water as expressed in terms of a water mobility value as a connecting substance. When an appropriate amount of the surplus water is contained in the heat generating composition, it is assumed that the surplus water causes hydration with a hydrophilic group in the components of the composition by a bipolar mutual action or hydrogen binding and is present in the surroundings of a hydrophobic group while having high structural properties. This surplus water is connecting water as a connecting substance for some meaning. Besides, there is water in a state called as free water. When the surplus water increases, the structure is softened, and free water is observed. Furthermore, in order that the iron powder causes an oxidation reaction, the existing amount of water and the feed amount of oxygen onto the surface of the iron powder become a control factor. It is said that the water is not sufficient for about an adsorbing water film (up to 100 angstroms), and an oxidation rate is small. When the adsorbing film is about 1 $\mu$m, not only the amount of water is sufficient, but also the feed of oxygen onto the surface of the iron powder is easy because the thickness of the water film is thin, thereby exhibiting a large oxidation rate. When the film becomes thicker and the adsorbing film becomes thick exceeding 1 $\mu$m, the feed amount of oxygen is reduced. As a result of obtaining knowledge that one expressing an optimal amount of water at which moldability and oxidation rate in fixed or higher levels are exhibited is a water mobility value and is from 0.01 to 20, the invention has been accomplished. That is, by using an appropriate amount of surplus water, the particles of the respective components are secured by a surface tension of the water. Thus, moldability is revealed in the heat generating composition, and the water does not function as a barrier layer. Accordingly, the moldable heat generating composition to be used in the invention comes into contact with air to cause heat generation. Furthermore, the microheater has an exothermic part as produced by laminating the heat generating composition molded body resulting from molding the moldable heat generating composition on a substantially planar substrate, further covering the covering material on the heat generating composition molded body and then heat sealing. It is possible to provide a microheater which is able to cause heat generation without moving water in the heat generating composition molded body as produced by a lamination system to the packaging material or water absorptive sheet, has flexibility by itself, is excellent in wearing on each place of a human body and places as required to have flexibility, such as materials having a curved surface and is excellent in feeling for use and a process for producing the same. Furthermore, among the substrate, covering material and heat generating composition molded body, by temporarily adhering at least the covering material and the heat generating composition molded body via a sticky layer and then heat sealing the surroundings of the heat generating molded body and the surroundings of the microheater, it is possible to design to achieve high-speed production of a microheater. In this way, a microheater in which the heat generating composition molded body and the covering material are temporarily adhered to each other via a sticky layer is obtained.

[Best Modes for Carrying Out the Invention]

The microheater of the invention is a microheater having a heat generating composition molded body made of a moldable heat generating composition containing surplus water as a connecting substance accommodated in an air-permeable accommodating bag, which is characterized in that:

1) the accommodating bag is made of a heat seal layer-containing substrate and a covering material and has an exothermic part as formed by laminating a heat generating composition molded body as molded on the substrate which is substantially planar and does not have a pocket, an accommodating division and an accommodating section, covering by the covering material and heat sealing the periphery of the heat generating composition molded body, 2) the moldable heat generating composition contains, as essential components, an iron powder, a carbon component, a reaction accelerator and water, has a content of water in the moldable heat generating composition of from 1 to 60 %, does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air,

3) a volume of the heat generating composition molded body is from 0.1 to 30 cm$^3$, and a capacity of the exothermic part to a ratio of the volume of the heat generating composition molded body is from 0.6 to 1.0, and
4) a maximum height of the exothermic part is from 0.1 to 10 mm.
Incidentally, in the invention, the heat generating composition molded body may be compressed, and a heat generating composition compressed body as its compressed body is also included in the heat generating composition molded body.

A flocculant aid, a flocculant, an agglomeration aid, a dry binding material, a dry binding agent, a dry binder, a sticky raw material, a thickener and an excipient are noxious to the exothermic reaction. What a pocket is provided in the substrate makes molding complicated and is problematic in view of costs. The invention does not use them and solves these problems at once.

In the structure of the microheaters, the heat generating composition molded body is accommodated between at least two opposing surfaces; the surroundings of the heat generating composition molded body are sealed; at least one surface is permeable to oxygen; and when the heat generating composition molded body which is a molded body of a moldable heat generating composition is sealed by two surfaces, the structure has a capacity of the heat generating composition molded body, a capacity of a space and a capacity of an exothermic part. In the invention, it is only required that the packaging material which constitutes each surface is constituted of a substrate and a covering material and that at least one or a part thereof is permeable to air. The packaging material on which the heat generating composition molded body resulting from molding the moldable heat generating composition is laminated is called as the "substrate". The packaging material which is covered on the substrate and the heat generating composition molded body after the lamination is called as the "covering material". Furthermore, the substrate on which at least the heat generating composition molded body is substantially planar and does not have a pocket for accommodating the heat generating composition molded body.

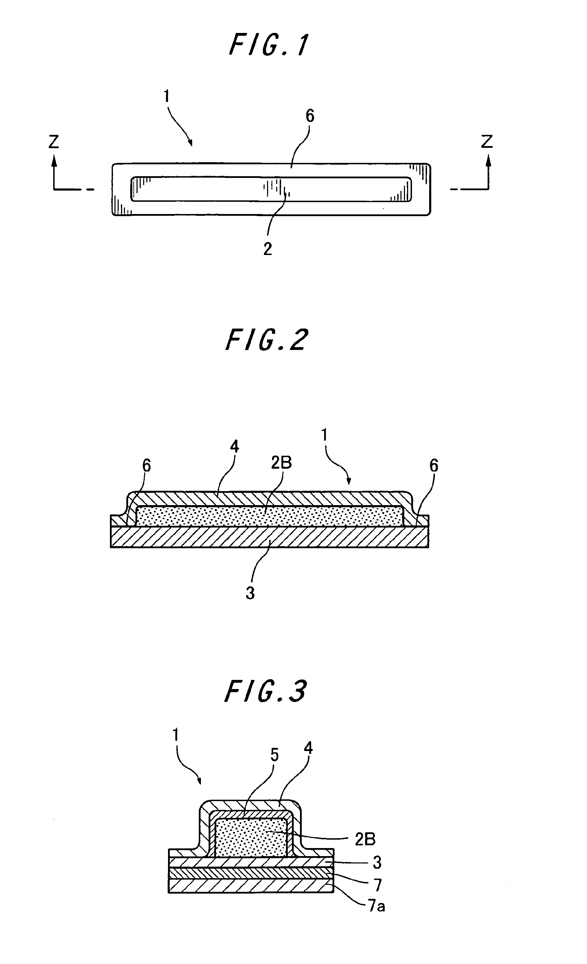

The "pocket" as referred to herein is an accommodating pocket and is a pocket in which the heat generating composition molded body is accommodated as described in JP-T-11-508786. Since irregularities which are not used for accommodating the heat generating composition molded body are not a pocket, even when such irregularities are present in a substrate, such a substrate is to be defined as a substantially planar substrate. The method of the invention and a method of using an accommodating pocket will be described as follows. As shown in Fig. 2, a substrate 3 of the invention is substantially planar. A heat generating composition molded body is laminated on the substrate, a covering material is further covered thereon, and the surroundings of the heat generating composition molded body are sealed. On the other hand, in the method of using an accommodating pocket, an accommodating pocket is previously prepared in the substrate, a granular heat generating composition is filled therein, and another packaging material is further covered thereon, followed by sealing.

In the case of a molding system of the invention, with respect to the molding order, the size of the heat generating composition molded body is determined, and the size of the exothermic part is then determined.

The fixing measure of the invention is not limited so far as it has a fixing ability such that a microheater or a material having an exothermic part can be fixed in a required part.
As the fixing measure, generally employed fixing measures such as an adhesive layer, a hook and eye, a hook and button, a hook and loop fastener such as Velcro, a magnet, a belt, a string, and a combination thereof can be arbitrarily used.
Incidentally, in the case of a belt, a fixing measure for adjustment may further be constituted of a combination of a hook and loop fastener and an adhesive layer.
Here, the "hook and loop fastener" as referred to herein has a fastening function by a combination of a loop as a female fastener with a male fastener capable of fastening the female fastener thereto, which is known as trade names such as Magic Tape (a registered trademark), Magic Fastener (a registered trademark), Velcro Fastener, and Hook and Loop Tape. Examples of the material having a loop function include non-woven fabrics and woven fabrics of napped or hole-containing yarns. Such a material having a loop function (female fastener function) may be covered on the surface of a paddling forming the band, or the band may be constructed of such a material itself. Although the hook member which is the male fastener member is not particularly limited, examples thereof include hook members formed of a polyolefin based resin (for example, polyethylene and polypropylene), a polyamide, a polyester, etc. Although the shape of the hook is not particularly limited, a hook having a cross-sectional shape such as an I type, an inverted L type, an inverted J type, and a so-called mushroom type is preferable because it is easily hooked by the loop and does not give an extreme stimulus to the skin. Incidentally, the hook may be adhered to the entire area of a fastening tape, and only the hook may be used as a fastening tape while omitting a tape substrate.
The adhesive layer may contain at least one member selected from additional components consisting of a water retaining agent, a water absorptive polymer, a pH adjusting agent, a surfactant, an organosilicon compound, a hydrophobic polymer compound, a pyroelectric substance, an antioxidant, an aggregate, a fibrous material, a moisturizer, a functional substance, and a mixture thereof.

The adhesive constituting the adhesive layer is not limited so far as it has an adhesive strength necessary for adhering to the skin or clothes. Adhesives of every form such as a solvent based adhesive, an aqueous adhesive, an emulsion type adhesive, a hot melt type adhesive, a reactive adhesive, and a pressure sensitive adhesive are employable.

The adhesive layer includes a non-hydrophilic adhesive layer constituted of a non-hydrophilic adhesive and a non-hydrophilic adhesive layer constituted of a non-hydrophilic adhesive.

It is to be noted that a material whose water absorption properties are improved by containing a water absorptive polymer or a water retaining agent in the non-hydrophilic adhesive layer is dealt as the non-hydrophilic adhesive layer.

A hot melt based adhesive may be provided between the non-hydrophilic adhesive layer and the substrate or the covering material.

Furthermore, in the case where the hydrophilic adhesive layer is provided in a microheater, there is no limitation. After a seal treatment of the microheater, an adhesive layer may be provided in the microheater.

Furthermore, the adhesive layer may air permeability or may not have air permeability. It may be properly selected depending upon the utility. With respect to the air permeability, the adhesive layer may be air-permeable as a whole. Examples thereof include an adhesive layer having air permeability as a whole of a region in which an adhesive is partially present and a portion where no adhesive is present is partially present.

In laminating an adhesive on an air-permeable substrate and/or a covering material in a stratiform state as it is, examples of a method for keeping its air permeability include a method in which an adhesive layer is partially laminated by printing or transferring an adhesive, thereby forming a non-laminated part as an air-permeable part; a method in which an adhesive is transferred in one direction while drawing a circle in a filament-like form or properly moved in the two-dimensional directions by transferring in a zigzag manner, whereby a space of the filament-like adhesive keeps air permeability or moisture permeability or the adhesive is foamed; and a method for forming a layer by a melt blow system.

Examples of the adhesive which constitutes the non-hydrophilic adhesive layer include acrylic adhesives, polyvinyl acetate based adhesives (for example, vinyl acetate resin based emulsions and ethylene-vinyl acetate resin based holt melt adhesives), polyvinyl alcohol based adhesives, polyvinyl acetal based adhesives, vinyl chloride based adhesives, polyamide based adhesives, polyethylene based adhesives, cellulose based adhesives, chloroprene (neoprene) based adhesives, nitrile rubber based adhesives, polysulfide based adhesives, polyurethane based adhesives, butyl rubber based adhesives, silicone rubber based adhesives, styrene based adhesives (for example, styrene based hot melt adhesives), rubber based adhesives, and silicone based adhesives. Of these, rubber based adhesives, acrylic adhesives, and adhesives containing a hot melt based polymer substance are desired for the reasons that they are high in the adhesive strength, are cheap, are good in long-term stability, and are small in reduction of the adhesive strength even by providing heat.

In addition to the base polymer, if desired, the adhesive may be compounded with other components such as tackifiers (for example, petroleum resins represented by rosins, chroman-indene resins, hydrogenated petroleum resins, maleic anhydride-modified rosins, rosin derivatives, and C-5 based petroleum resins), phenol based tackifiers (especially, tackifiers having an aniline point of not higher than 50 °C; for example, terpene phenol based resins, rosin phenol based resins, and alkylphenol based resins), softeners (for example, coconut oil, castor oil, olive oil, camellia oil, and liquid paraffin), softeners, anti-aging agents, fillers, aggregates, adhesion adjusting agents, adhesion modifiers, coloring agents, anti-foaming agents, thickeners, and modifiers, thereby improving performance such as an improvement in adhesion to nylon-made clothes and mixed yarn clothes.

Examples of the hot melt based adhesive include known hot melt based adhesives imparted with adhesion. Specific examples thereof include styrene based adhesives, polyester based adhesives made of, as a base polymer, a polyester, acrylic adhesives made of, as a base polymer, an acrylic resin, polyolefin based adhesives made of, as a base polymer, a polyolefin (for example, polyethylene, super low density polyethylene, polypropylene, ethylene-$\alpha$-olefin copolymers, and ethylene-vinyl acetate copolymers), and 1,2-polybutadiene based adhesives made of, as a base polymer, 1,2-polybutadiene; adhesives made of a modified body of the foregoing adhesive whose adhesion is improved or whose stability is changed; and mixtures of two or more kinds of these adhesives. Adhesive layers constituted of an expanded adhesive and adhesive layers constituted of a crosslinked adhesive can also be employed.

The non-aromatic hot melt based adhesive is not limited so far as it is made of, as a base polymer, a hot melt based adhesive not containing an aromatic ring. Examples thereof include olefin based hot melt based adhesives and acrylic hot melt based adhesives. As the non-aromatic polymer which is the base polymer not containing an aromatic ring, there are enumerated polymers or copolymers of an olefin or a diene. Examples thereof include olefin polymers. The olefin polymer includes polymers or copolymers of ethylene or an $\alpha$-olefin. Also, polymers resulting from adding a diene (for example, butadiene and isoprene) as other monomer thereto may be employed.

The $\alpha$-olefin is not limited so far as it is a monomer having a double bond in the terminal thereof. Examples thereof include propylene, butene, heptene, hexene, and octene.

The "aromatic hot melt based adhesive" as referred to herein means a hot melt based adhesive whose base polymer contains an aromatic ring. Examples thereof include styrene based hot melt based adhesives represented by A-B-A type block copolymers.

In the foregoing A-B-A type block copolymers, the A block is a non-elastic polymer block made of a monovinyl substituted aromatic compound such as styrene and methylstyrene; and the B block is an elastic polymer block made of a conjugated diene such as butadiene and isoprene. Specific examples thereof include a styrene-butadiene-styrene block copolymer (SBS), a styrene-isoprene-styrene block copolymer (SIS), and hydrogenated types thereof (for example, SEBS and SIPS). Mixtures thereof may also be used.

As a countermeasure for preventing a lowering of adhesive strength caused due to an increase of water of the non-hydrophilic adhesive layer, an adhesive layer obtained by further blending a water absorptive polymer in the non-hydrophilic adhesive can be used.

The hydrophilic adhesive which constitutes the hydrophilic adhesive layer is not particularly limited so far as it contains a hydrophilic polymer or a water-soluble polymer as the major component, has adhesion and is hydrophilic as an adhesive. Examples of the constitutional components of the hydrophilic adhesive include hydrophilic polymers (for example, polyacrylic acid), water-soluble polymers (for example, poly(sodium acrylate) and polyvinylpyrrolidone), crosslinking agents (for example, dry aluminum hydroxide and meta-silicic acid aluminic acid metal salts), softeners (for example, glycerin and propylene glycol), higher hydrocarbons (for example, light liquid paraffin and polybutene), primary alcohol fatty acid esters (for example, isopropyl myristate), silicon-containing compounds (for example, silicone oil), fatty acid glycerin esters (for example monoglycerides), oily components (for example, vegetable oils such as olive oil), antiseptics (for example, methyl p-hydroxybenzoate and propyl p-hydroxybenzoate), solubilizing agents (for example, N-methyl-2-pyrrolidone), thickeners (for example, carboxymethyl cellulose), surfactants (for example, polyoxyethylene hardened castor oil and sorbitan fatty acid esters), hydroxycarboxylic acid (for example, tartaric acid), excipients (for example, light silicic anhydride, water absorptive polymers, and kaolin), moisturizers (for example, D-sorbitol), stabilizers (for example, sodium edetate, p-hydroxybenzoic acid esters, and tartaric acid), crosslinking type water absorptive polymers, boron compounds (for example, boric acid), and water. They may be constituted as an arbitrary combination.

Furthermore, in the case where an adhesive layer is employed as the hydrophilic adhesive layer, if there is a difference in a water retaining force between the hydrophilic adhesive layer and the heat generating composition molded body, transfer of water occurs via a packaging material present therebetween such as a substrate, thereby causing inconveniences against the both. In particular, the transfer of water often occurs during the storage. In order to prevent this, it is preferable to provide a moisture-proof packaging material present therebetween. By using this, in the case where the microheater is accommodated in an outer bag as an air-impermeable accommodating bag and stored, the transfer of water can be prevented.

In the case where a hydrophilic adhesive layer is used as the adhesive layer, the moisture permeability of a moisture-proof packaging material as provided between the heat generating composition molded body and the hydrophilic adhesive layer is not limited so far as the transfer of water can be prevented within the range where the exothermic performance is not affected. The moisture permeability according to the Lyssy method is usually not more than 2 $g/m^2$/day, preferably not more than 1.0 $g/m^2$/day, more preferably not more than 0.5 $g/m^2$/day, and further preferably from 0.01 to 0.5 $g/m^2$/day. These values are a value under a condition under an atmospheric pressure at 40 °C and 90 % RH. Incidentally, the moisture-proof packaging material can be used as a substrate or a covering material and may be laminated singly on a substrate, a covering material, or the like.

Examples of the moisture-proof packaging material include metal vapor deposited films, vapor deposited films of a metal oxide, metal foil-laminated films, EVOH (ethylene/vinyl alcohol copolymer or ethylene/vinyl acetate copolymer saponified product) based films, biaxially stretched polyvinyl alcohol films, polyvinylidene chloride coated films, polyvinylidene chloride coated films obtained by coating polyvinylidene chloride on a substrate film (for example, polypropylene), metal foils such as an aluminum foil, air-impermeable packaging materials obtained by vapor depositing or sputtering a metal (for example, aluminum) on a polyester film substrate, and packaging laminates using a transparent barrier film of a structure in which silicon oxide or aluminum oxide is provided on a flexible plastic substrate. The air-impermeable packaging materials which are used in the outer bag, etc. can also be used.

Furthermore, packaging materials such as moisture-proof packaging materials as described in JP-A-2002-200108, the disclosures of which can be incorporated herein by reference, can be used.

In the case of using a water-containing hydrophilic adhesive (for example, a gel) in the adhesive layer, in order to adjust the moisture equilibrium between the heat generating composition and the adhesive layer, the content of a reaction accelerator (for example, sodium chloride) or a substance having a water holding power (for example, a water absorptive polymer) in the heat generating composition may be adjusted within the range of from 10 to 40 % by weight, preferably from 15 to 40 % by weight, and more preferably from 15 to 30 % by weight based on the heat generating composition. Furthermore, as the adhesive having good moisture permeability and low stimulation to the skin, water-containing adhesives (for example, hydrophilic adhesives and gels) as described in JP-A-10-265373 and JP-A-9-87173, adhesives which can be subjected to hot melt coating as described in JP-A-6-145050 and JP-A-6-199660, and rubber based adhesives as described JP-A-10-279466 and JP-A-10-182408, the disclosures of which are totally incorporated herein by reference, are useful.

The functional substance which is contained in the adhesive layer is not limited so far as it is a substance having any function. There can be enumerated at least one member selected from aromatic compounds, vegetable extracts, crude drugs, perfumes, slimming agents, analgesics, blood circulation promoters, swelling improvers, antibacterial agents, sterilizers, mold inhibitors, odor eaters, deodorants, percutaneously absorptive drugs, fat-splitting components, minus ion generators, far infrared ray radiants, magnetic bodies, fomentations, cosmetics, bamboo vinegar, and wood vinegar. Specific examples thereof include aromatic compounds (for example, menthol and benzaldehyde), vegetable extracts (for example, mugwort extract), crude drugs (for example, moxa), perfumes (for example, lavender and rosemary), slimming agents (for example, aminophylline and tea extract), analgesic drugs (for example, indomethacin and dl-camphor), blood circulation promoters (for example, acidic mucopolysaccharide and chamomile), swelling improvers (for example, horse chestnut extract and flavone derivatives), fomentations (for example, aqueous boric acid, physiological saline, and aqueous alcohols), fat-splitting components (for example, jujube extract, caffeine, and tonalin), cosmetics (for example, aloe extracts, vitamin preparations, hormone preparations, anti-histamines, and amino acids), antibacterial agents and sterilizers (for example, carbolic acid derivatives, boric acid, iodine preparations, invert soaps, salicylic acid based substances, sulfur, and antibiotics), and mold inhibitors.

The percutaneously absorptive drug is not particularly limited so far as it has percutaneous absorption. Examples thereof include corticosteroids, anti-inflammatory drugs, hypertension drugs, anesthetics, hypnotic sedatives, tranquillizers, antibacterial substances, antifungal substances, skin stimulants, inflammation inhibitors, anti-epileptics, analgesics, antipyretics, anesthetics, mold inhibitors, antimicrobial antibiotics, vitamins, antiviral agents, swelling improvers, diuretics, antihypertensives, coronary vasodilators, anti-tussive expectorants, slimming agents, anti-histamines, antiarrhythmic agents, cardiotonics, adrenocortical hormones, blood circulation promoters, local anesthetics, fat-splitting components, and mixtures thereof. However, it should not be construed that the invention is limited thereto. These drugs are used singly or in admixture of two or more kinds thereof as the need arises.

The content of such a functional substance is not particularly limited so far as it falls within the range where the effect of a medicine can be expected. However, from the viewpoints of adhesive strength as well as pharmacological effect and economy, the content of the functional substance is preferably from 0.01 to 25 parts by weight, and more preferably from 0.5 to 15 parts by weight based on 100 parts by weight of the adhesive.

Furthermore, a method for providing the adhesive layer is not limited so far as a thermal packaging body for joint surroundings can be fixed. The adhesive layer may be entirely provided or partially or intermittently provided. Examples of its shape include various shapes such as a network-like shape, a stripe-like shape, a dot-like shape, and strip-like shape.

Incidentally, though the temperature of the microheater as thermal moxibustion goods is not limited, it is preferably 40 °C or higher, more preferably from 40 to 65 °C, further preferably from 40 to 50 °C, still further preferably from 40 to 45 °C, and even further preferably from 40 to 43 °C.

Furthermore, a time for keeping 40 °C or higher is preferably from one minute to 5 hours, more preferably from one minute to 4 hours, further preferably from 30 minutes to 4 hours, and still further preferably from one hour to 4 hours.

The microheaters of the invention can be chained to form a large-sized microheater or can be easily incorporated in a packaging material for fixing the body or the like. Such becomes a microheater which is fitted to the body shape, imparts a comfortable feeling and is excellent in feeling for use.

In the sectional exothermic part or the heat generating composition molded body of the invention, its maximum width is usually from 0.5 to 60 mm, preferably from 0.5 to 50 mm, more preferably from 1 to 50 mm, further preferably from 3 to 50 mm, still further preferably 3 to 30 mm, even further preferably from 5 to 20 mm, even still further preferably from 5 to 15 mm, and most preferably from 5 to 10 mm. Furthermore, its maximum height is usually from 0.1 to 30 mm, preferably from 0.1 to 10 mm, more preferably from 0.3 to 10 mm, further preferably from 1 to 10 mm, and still further preferably from 2 to 10 mm. Moreover, its longest length is usually from 5 to 300 mm, preferably from 5 to 200 mm, more preferably from 5 to 100 mm, further preferably from 20 to 150 mm, and still further preferably from 30 to 100 mm.

A capacity of the sectional exothermic part or a volume of the heat generating composition molded body is usually from 0.015 to 500 $cm^3$, preferably from 0.04 to 30 $cm^3$, more preferably from 0.1 to 30 $cm^3$, further preferably from 1 to 30 $cm^3$, and still further preferably from 3 to 20 $cm^3$.

In the sectional exothermic part, when the sectional exothermic part which is an accommodating region of the heat generating composition is filled with the heat generating composition molded body, a volume ratio of the volume of the heat generating composition molded body which is an occupying region of the heat generating composition molded body to the capacity of the sectional exothermic part which is an accommodating region of the heat generating composition is usually from 0.6 to 1, preferably from 0.7 to 1, more preferably from 0.8 to 1, and further preferably from 0.9 to 1.0.

Furthermore, a width of the sectioned part which is a space between the sectional exothermic parts is not limited so far as sectioning can be achieved. It is usually from 0.1 to 50 mm, preferably from 0.3 to 50 mm, more preferably from 0.3 to 50 mm, further preferably from 0.3 to 40 mm, still further preferably from 0.5 to 30 mm, even further preferably from 1.0 to 20 mm, and even still further preferably from 3 to 10 mm.

Incidentally, the heat generating composition molded body or the sectional exothermic part may have any shape. The shape may be a planar shape, and examples thereof include a circular shape, an elliptical shape, a polygonal shape, a star shape, and a flower shape. Also, the shape may be a three-dimensional shape, and examples thereof include a polygonal pyramidal shape, a conical shape, a frustum shape, a spherical shape, a parallelepiped shape, a cylindrical shape, a semi-pillar shape, a semicylindroid shape, a semicylidrical shape, a pillar shape, and a cylindroid shape. Furthermore, in these shapes, the corner may be rounded, thereby processing the corner in a curvilinear or curved state, or the central part may be provided with a concave.

Furthermore, the "volume of the heat generating composition molded body of the invention" as referred to herein means a volume of the heat generating composition molded body or compressed heat generating composition molded body. Furthermore, the "capacity of the sectional exothermic part" as referred to herein means an internal capacity of the sectional exothermic part having a heat generating composition molded body accommodated therein.

The shape of the microheater may be any shape and can be selected from the group consisting of a rectangular shape, a circular shape, an elliptical shape, a polygonal shape, a broad bean-like shape, an eye mask-like shape, a paper lantern-like shape, a cocoon-like shape, a gourd-like shape, a rectangular shape with rounded corners, a square shape with rounded corners, an egg-like shape, a boomerang-like shape, a comma-shaped bead-like shape, a wing-like shape, a nose-like shape, a star-like shape, and a foot-like shape.

Furthermore, the microheater or accommodating bag can be provided with at least one member of characters, designs, symbols, numerals, patterns, photographs, pictures, and colors in at least a part thereof.

In sealing, there is no limitation with respect to the sealing method so far as sealing can be carried out. A sealing method is properly selected depending upon the desire. For example, as one example thereof, sealing is carried out in a point-like (intermittent) manner or entirely by contact bonding seal (adhesive seal), warm contact bonding seal (adhesive seal), bonding seal, heat bonding seal, heat melt seal (heat seal), etc. by means of pressurizing, warming, heating or a combination thereof via an adhesive layer and/or a bonding agent layer and/or a heat seal layer. Selection of any one or a combination of these methods may be made depending upon the desire. In this way, it is possible to seal and form an exothermic part. Sewing processing can also be employed as one of seal means. Above all, heat sealing is preferable in view of the strength. In particular, in the case of heat sealing at a high speed, a method in which contact bonding sealing using an adhesive layer is employed for temporary adhesion and thereafter, heat sealing is carried out is preferable from the standpoint of securing heat sealing.

In the substrate or the substrate for forming an accommodating bag such as a covering material, the seal width of the periphery to be sealed can be properly determined. The seal width is usually not more than 50 mm, preferably from 1 to 30 mm, more preferably from 3 to 20 mm, and further preferably from 5 to 20 mm.

Furthermore, at least a part of the surface of the heat generating composition molded body may be covered by an air-permeable adhesive layer such as a netlike polymer, or an underlay material such as non-woven fabrics may be provided between the air-permeable adhesive layer and the covering material.

Furthermore, the entire surface or its part of at least one member of the heat generating composition molded body, the substrate, the covering material, the air-permeable adhesive layer and the underlay material may be subjected to a pressurizing treatment or the like or may be provided with irregularities. In this way, the transfer of the laminate between the substrate and the covering material may also be prevented.

That is, a material prepared by appropriately compressing the heat generating composition molded body which is a molded material of the heat generating composition of the invention by pressurizing is markedly improved in moldability. For example, even when a perforated film which is difficult with respect to the pressure adjustment is used as a raw material of the air-permeable part in place of the porous film, or even when an inner pressure of the accommodating bag becomes equal to or more than the outer pressure, shape collapse hardly occurs so that the use of a porous film and a perforated film is possible. Accordingly, not only the range for selecting an air-permeable raw material is widened so that the costs can be lowered, but also a body to be warmed can be uniformly warmed at an appropriate temperature over a long period of time.

For the purpose of containing a magnetic substance in the exothermic part to improve the blood circulation or stiff shoulders due to a magnetic effect, it is also possible to accommodate therein a magnetic substance such as a magnet.

In this way, in sticking the microheater to an affected part such that a muscle or tendon of the affected part is in parallel to the adhesive plaster body, a reverse physical tension is continuously given to the tension of the muscle or tendon, whereby the tension of the muscle or tendon is relieved. Furthermore, since a fault strain is brought between the adjacent muscles or tendons and the tension of the muscles or tendons is relieved, the physical tension is reinforced. As a result, residence of vital energy and blood is dissolved, and a symptom of menstrual pain is lightened. Furthermore, a stimulus of a so-called "acupuncture point" which is a regional stimulus is also effective for relieving the symptom of menstrual pain.

The microheater is accommodated in an air-impermeable accommodating bag, stored and transported. Examples thereof include a microheater prepared by interposing a produced microheater between two air-impermeable films or sheets, punching the two sheets or sheets into a size larger than that of the microheater at the same time with or after this interposition, and sealing the two films or sheets in the surroundings exceeding the size of the microheater at the same time with or after this punching.

Though the microheater is accommodated in an air-impermeable accommodating bag, stored and transported, the outer bag is not limited so far as it is impermeable to air, and it may be made of a laminate.

The microheater of the invention is able to give various shapes, thicknesses and temperature zones and therefore, can be used as a sticking agent other than those for usual warmth taking of the body. That is, the microheater of the invention can be used for various utilities such as use for thermal moxibustion, use for inner footwear of foot, etc., use for a joint, facial esthetic use, use for eyes, use for a wet compress pack, use for a medical body warmer, use for a neck, use for a waist, use for a mask, use for a glove, use for hemorrhage, use for relieving a symptom of menstrual pain, use for shoulders, use for a cushion, use for an aroma, use for an abdomen, use for absorption of oxygen, and use for remedy of cancer. In addition, the microheater of the invention can be used for heating or warming machines, pets, etc.

Furthermore, as a method for use of the microheater, for example, there is enumerated a method for use in which the microheater is applied in a site of the body having a pain of a person who needs the remedy, the temperature of the skin and the time for keeping are properly chosen depending upon the person who needs the remedy, and an acute, recurrent or chronic muscular pain, a skeletal pain or a related pain is remedied such that the suffering is comfortably and substantially relieved.

A raw material of the substrate or covering material is not limited so far as it functions as an accommodating bag of the heat generating composition. Usually, raw materials which are used in chemical body warmers or heat generating bodies can be used. Examples of the raw material include air-impermeable raw materials, air-permeable raw materials, water absorptive raw materials, non-water absorptive raw materials, non-extensible raw materials, extensible raw materials, stretchable raw materials, non-stretchable raw materials, foamed raw materials, non-foamed raw materials, non-heat sealable raw materials, and heat sealable raw materials. The raw material can be properly used depending upon a desired utility in a desired form such as films, sheets, non-woven fabrics, woven fabrics, and composites thereof. In general, the substrate is made of an air-impermeable film or sheet, and the covering material is made of an air-permeable film or sheet or non-woven fabric, and vice versa. The both may be air-permeable. As the underlay material, an air-permeable underlay material and an air-impermeable underlay material may be used for different purposes.

The packaging material of the accommodating bag may be of a single-layered structure or multilayered structure, and its structure is not limited. Furthermore, though the packaging material is composed of at least a substrate and a covering material, a packaging material for laminating the heat generating composition molded body is the substrate, and a packaging material for covering on the heat generating composition molded body is the covering material regardless of whether the packaging material is air-permeable or air-impermeable. An embodiment of a multilayered structure in which an air-impermeable packaging material is the substrate and an air-permeable packaging material is the covering material will be hereunder described as one example. That is, in this embodiment, the substrate is made of layer A/layer B, layer A/layer B/layer C, or layer A/layer B/layer C/layer D; and the covering material is made of layer F/layer G, layer E/layer F/layer G, or layer F/layer H/layer G. Examples of the layer A include thermoplastic resin films (for example, polyethylene), heat seal layers (for example, polyethylene and EVA), and water absorptive papers; examples of the layer B include non-woven fabrics of a thermoplastic resin (for example, nylons), non-water absorptive papers, water absorptive papers, thermoplastic resin films (for example, polyethylene films, polypropylene films, polyester films, and polyamide (for example, nylons) films), wicks (for example, non-water absorptive papers and water absorptive papers); examples of the layer C include adhesive layers, non-water absorptive papers, water absorptive papers, thermoplastic resin films (for example, polyethylene), non-slip layers, and non-woven fabrics of a thermoplastic resin (for example, polyesters and nylons); examples of the layer D include separators, thermoplastic resin films (for example, polyethylene), and non-woven fabrics; examples of the layer E include heat seal layers; examples of the layer F include porous films or perforated films made of a thermoplastic resin (for example, polyethylene), films made of a thermoplastic resin (for example, polyethylene), non-water absorptive papers, and water absorptive papers; examples of the layer G include non-woven fabrics of a thermoplastic resin (for example, polyesters and nylons) ; and examples of the layer H include non-water absorptive papers and water absorptive papers. Examples of the substrate or covering material include heat seal layer made of polyethylene obtained by using a metallocene catalyst/polypropylene film, polyethylene-made heat seal layer/polypropylene film, EVA-made heat seal layer/polypropylene film, EVA-made heat seal layer/polypropylene film/adhesive layer/separator, EVA-made heat seal layer/polyethylene film/nylon non-woven fabric, non-woven fabric/porous film, heat seal layer made of polyethylene obtained by using a metallocene catalyst/polyethylene film/nylon non-woven fabric, heat seal layer made of polyethylene obtained by using a metallocene catalyst/polypropylene film/polypropylene non-woven fabric, non-woven fabric/(paper and/or perforated (provided by a needle or laser) film)/porous film, non-woven fabric/(paper and/or porous film) /perforated (provided by a needle or laser) film, and non-woven fabric/(paper and/or porous film)/non-woven fabric. A method for laminating the respective layers is not limited. The respective layers may be directly laminated; the respective layers may be laminated via an air-permeable adhesive layer or a laminating agent layer; and the respective layers may be laminated by hot melt extrusion or the like. Furthermore, in the invention, it is to be noted that polyethylene produced by using a metallocene catalyst is also included in the polyethylene.

For example, in the case of laminating the foregoing raw material such as non-woven fabrics and porous films via an air-permeable sticky layer, examples of a method for forming the air-permeable sticky layer include a method in which a sticky substance is fibrillated by an appropriate system such as a curtain spray system, a melt blow system or a slot spray system for blowing and spreading a sticky substance via hot air under heat melting and spread and accumulated on an appropriate supporting substrate made of a porous film, an air-permeable substrate, a separator, etc., thereby forming a porous sticky layer.

A thickness of each of the substrate, the covering material, the underlay material, and the raw material constituting the same varies depending upon the utility and is not limited. The thickness is usually from 5 to 5,000 $\mu$m, preferably from 10 to 500 $\mu$m, and more preferably from 20 to 250 $\mu$m.

The air-impermeable raw material is not limited so far as it is air-impermeable. Examples thereof include films, sheets or coatings made of a polymer (for example, polyethylene, polypropylene, nylons, polyacrylates, polyesters, polyvinyl alcohols, and ethylene-vinyl acetate copolymers) and laminates thereof with a metal (including a semiconductor) compound (for example, silicon oxide) or composite raw materials using the same.

Of the foregoing air-impermeable raw materials, examples of a film having high air impermeability include films provided with a single layer or multiple layers of a thin film having a metal including a semiconductor or a compound thereof provided on an air-impermeable raw material film. Examples of the metal including a semiconductor include silicon, aluminum, and alloys or mixtures containing such a metal. Examples of the metal (including a semiconductor) compound include oxides, nitrides and oxynitrides of the foregoing metals or alloys or mixtures. Examples of the layer include silicon oxide layers, aluminum oxide layers, and silicon oxynitride layers; layers obtained by laminating an arbitrary layer of these layers on a polyester-made film; and layers obtained by further laminating a stretched polyolefin film (for example, a biaxially stretched polypropylene film) thereon.

The air-permeable raw material is not limited so far as it is air-permeable. Examples thereof include air-permeable films (for example, porous films and perforated films); materials having air permeability by themselves (for example, papers and non-woven fabrics); materials prepared by laminating at least one of papers and air-permeable films and non-woven fabrics so as to have air permeability; materials prepared by providing an air-impermeable packaging material comprising a non-woven fabric having a polyethylene film laminated thereon with fine pores by using a needle, etc. so as to have air permeability; non-woven fabric whose air permeability is controlled by laminating a fiber and heat bonding under pressure; porous films; and materials prepared by sticking a non-woven fabric onto a porous film. The "perforated film" as referred to herein is a film prepared by providing an air-impermeable film (for example, polyethylene films) with fine pores by using a needle so as to have air permeability.

The air permeability is not limited so far as the heat generation can be kept. In the case of use in usual heat generation, the air permeability is usually from 50 to 10, 000 g/m$^2$/24 hr, preferably from 70 to 5,000 g/m$^2$/24 hr, more preferably from 100 to 2, 000 g/m$^2$/24 hr, and further preferably from 100 to 700 g/m$^2$/24 hr in terms of moisture permeability by the Lyssy method.

When the moisture permeability is less 50 g/m$^2$/24 hr, the heat value is small and a sufficient thermal effect is not obtained, and therefore, such is not preferable. On the other hand, when it exceeds 10,000 g/m$^2$/24 hr, the exothermic temperature is high so that a problem in safety may possibly be generated, and therefore, such is not preferable. However, there is no limitation even when the moisture permeability exceeds 10,000 g/m$^2$/24 hr depending upon the utility, or even in the use at a moisture permeability closed to the open system, according to circumstances.

The stretchable packaging material is not particularly limited so far as it is stretchable. That is, it is only required that the stretchable packaging material is stretchable as a whole. The stretchable packaging material may be formed of a single material or a composite material of stretchable substrates or a combination of a stretchable substrate and a non-stretchable substrate.

Examples of the stretchable packaging material include single materials (for example, natural rubbers, regenerated rubbers, synthetic rubbers, elastomers, and stretchable shape memory polymers) and mixtures thereof, mixed materials or blended materials of such a stretchable raw material and a non-stretchable raw material or fabrics constituted of a combination of these materials, films, yarns, strands, ribbons, tapes, and stretchable films with a scrim structure.

The porous film is not limited and can be properly selected among porous films obtained by stretching a film made of a polyolefin based resin (for example, polyethylene, linear low density polyethylene, and polypropylene) or a fluorine based resin (for example, polytetrafluoroethylene) and a filler.

The non-woven fabric is not limited. Single non-woven fabrics of a single fiber or composite fiber made of a material such as rayon, nylons (polyamides), polyesters, polyacrylates, polypropylene, vinylon, polyethylene, polyurethane, cupra, cotton, cellulose, and pulp, or laminates of blended or accumulated fiber layers of such fibers are useful. Furthermore, from the standpoint of production process, dry non-woven fabrics, wet non-woven fabrics, spunbonds, spunlaces, and the like can be used. Non-woven fabrics made of a composite fiber having a core-sheath structure are also useful. A non-woven fabric in the side which is brought into contact with the skin is preferably a napping (fluffy) non-woven fabric. Also, stretchable non-woven fabrics and non-stretchable non-woven fabrics are useful.

The water absorptive raw material is not particularly limited so far as it is a water absorptive film or sheet.

The water absorptive raw material is not particularly limited so far as it has water absorption properties consequently regardless of whether or not the raw material has water absorption properties by itself.

Specific examples thereof include water absorptive foamed films or sheets having water absorption properties (for example, foamed bodies of water absorptive foamed polyurethane, etc.) or papers, non-woven fabrics or woven fabrics formed of a fiber having water absorption properties, non-woven fabrics or woven fabrics containing a fiber having water absorption properties, and water absorptive materials such as water absorptive porous films or sheets. Besides, there are enumerated materials in which regardless of the presence or absence of water absorption properties, a water absorbing agent is contained, impregnated, kneaded, transferred or carried on a foamed film or sheet, a non-woven fabric, a woven fabric or porous film or sheet, thereby imparting or increasing water absorption properties; and materials in which regardless of the presence or absence of water absorption properties, a water absorptive raw material such as water absorptive foamed films or sheets, papers, non-woven fabrics, woven fabrics, and porous films or sheets as cut in a planar shape according to the invention is attached to one side or both sides of the material according to the invention, thereby imparting water absorption properties.

In particular, in the heat generating body of the invention, for the purpose of forming the plane which is brought into contact with the skin into a comfortable plane by imparting water absorption properties against sweat, etc., in order that in the case of sweating, the sweat is absorbed, it is preferable that a packaging material in the plane which is brought into contact with the skin is constituted of a packaging material using a non-woven fabric or a woven fabric containing, as the major component, a water absorptive fiber having a water retention of 20 % or more. Examples of the water absorptive fiber having a water retention of 20 % or more include cottons, silks, hemps, wools, polyacrylonitrile based synthetic fibers, polyamide based synthetic fibers, polyvinyl alcohol based synthetic fibers, acetate fibers, triacetate fibers, and regenerated fibers. In addition, non-woven fabrics having a highly water absorptive polymer held in a non-woven fabric can be used as the non-woven fabric having excellent water absorption properties. Incidentally, non-woven fabrics or woven fabrics containing such a fiber as the major component are relatively good with respect to the feeling against the skin.

In addition, highly water absorptive packaging materials having high absorption properties of sweat can be used as the packaging material. Examples thereof include non-woven fabrics containing a fiber whose surface is coated with a highly water absorptive resin, non-woven fabrics containing a hollow fiber having a number of fine pores on the surface thereof, and non-woven fabrics containing a fiber having a capillary action by forming a number of pouches or plural layers in the cross-sectional shape.

Besides, non-woven fabrics or films having a water absorptive inorganic compound held on a non-sticky surface of a packaging material can be used. Examples thereof include non-woven fabrics resulting from holding a powder (for example, diatomaceous earth, zeolite, and silica gel) on a non-woven fabric and films resulting from holding a relatively large amount of a powder (for example, silica and alumina) on a synthetic resin (for example, polyethylene).

The outer bag is not limited so far as it is impermeable to air, and it may be made of a laminate. Examples thereof include nylon, polyester and polypropylene films which are subjected to a moisture-proof treatment with OPP, CPP, polyvinylidene chloride, metal oxides (including semiconductors) such as aluminum oxide and silicon oxide, etc., aluminum foils, and aluminum-deposited plastic films. As one example thereof, there is enumerated a microheater in which the produced microheaters is sealed and fixed between two air-impermeable films or sheets.

The moldable heat generating composition is not limited so far as it is a heat generating composition which contains, as essential components, an iron powder, a carbon component, a reaction accelerator and water, does not contain a flocculant aid, a flocculant, an agglomeration aid, a dry binding material, a dry binding agent, a dry binder, an adhesive binder, a thickener and an excipient, contains surplus water so as to have a water mobility value of from 0.01 to 20, has moldability due to the surplus water, with the water in the heat generating composition not functioning as a barrier layer, and is capable of causing an exothermic reaction upon contact with air.

Incidentally, in the invention, what water does not function as a barrier layer and causes an exothermic reaction upon contact with air means that water in a heat generating composition does not function as a barrier layer which is an air intercepting layer and immediately after the production of a heat generating composition, comes into contact with air, thereby immediately causing an exothermic reaction.

The heat generating composition is not limited so far as it is a heat generating composition which contains, as essential components, an iron powder, a carbon component, a reaction accelerator and water, does not contain a flocculant aid, a flocculant, an agglomeration aid, a dry binding material, a dry binding agent, a dry binder, an adhesive binder, a thickener and an excipient, contains surplus water so as to have a water mobility value of from 0.01 to 20, has moldability due to the surplus water, with the water in the heat generating composition not functioning as a barrier layer, and is capable of causing an exothermic reaction upon contact with air.

In addition, if desired, at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof may be further added to the heat generating composition.

Furthermore, in the heat generating composition of the invention or the like, although there is no particular limitation for the compounding ratio thereof, it is preferred to select the compounding ratio such that the amount of the reaction accelerator is from 1.0 to 50 parts by weight, the amount of water is from 1.0 to 60 parts by weight, the amount of the carbon component is from 1.0 to 50 parts by weight, the amount of the water retaining agent is from 0.01 to 10 parts by weight, the water absorptive polymer is from 0.01 to 20 parts by weight, the amount of the pH adjusting agent is from 0.01 to 5 parts by weight, and the amount of the hydrogen formation inhibitor is from 0.01 to 12 parts by weight, respectively based on 100 parts by weight of the iron powder; and that the heat generating composition has a water mobility value of from 0.01 to 20.

In addition, the following components may be added in compounding ratios as described below to the iron powder to the heat generating composition. That is, the amount of the metal other than iron is from 1.0 to 50 parts by weight, the amount of the metal oxide other than iron oxide is from 1.0 to 50 parts by weight, the amount of the surfactant is from 0.01 to 5 parts by weight, the amount of each of the hydrophobic polymer compound, the aggregate, the fibrous material, the functional substance, the organosilicon compound and the pyroelectric substance is from 0.01 to 10 parts by weight, the amount of each of the moisturizer, the fertilizer component and the heat generating aid is from 0.01 to 10 parts by weight, and the amount of the acidic substance is from 0.01 to 1 part by weight based on 100 parts by weight of the iron powder. Incidentally, a magnetic material may further be compounded, and its compounding ratio may be properly determined depending upon the desire.

Incidentally, these compounding ratios can also be applied in a reaction mixture and a heat generating mixture. Furthermore, a water mobility value of the reaction mixture is usually less than 0.01.

As the water, one from a proper source may be employed. Its purity and kind and the like are not particularly limited.

In the case of the heat generating composition, the content of water is preferably from 1 to 70 % by weight, more preferably from 1 to 60 % by weight, further preferably from 7 to 60 % by weight, still further preferably from 10 to 50 % by weight, and even further preferably from 20 to 50 % by weight of the heat generating composition.

Furthermore, in the case of the reaction mixture or heat generating mixture prior to the contact treatment with an oxidizing gas, the content of water is preferably from 0.5 to 20 % by weight, more preferably from 1 to 20 % by weight, further preferably from 3 to 20 % by weight, and still further preferably from 4 to 15 % by weight of the reaction mixture or heat generating mixture.

The carbon component is not particularly limited so far as it contains carbon as a component. Examples thereof include carbon black, graphite, active carbon, carbon nanotubes, carbon nanohorns, and flullerenes. Carbon which has become conductive by doping or the like is also employable. There are enumerated active carbons as prepared from coconut shell, wood, charcoal, coal, bone carbon, etc. and carbons as prepared from other raw materials such as animal products, natural gases, fats, oils, and resins. In particular, active carbons having an adsorption retaining ability are preferable.

Furthermore, it is not always required that the carbon component is present alone. In the case where an iron powder containing the carbon component and/or covered by the carbon component is used in the heat generating composition, it is to be noted that the heat generating composition contains the carbon component even though the carbon component is not present alone.

The reaction accelerator is not particularly limited so far as it is able to promote the reaction of the heat generating substance. Examples thereof include metal halides, nitrates, acetates, carbonates, and metal sulfates. Examples of metal halides include sodium chloride, potassium chloride, magnetic chloride, calcium chloride, ferrous chloride, ferric chloride, sodium bromide, potassium bromide, ferrous bromide, ferric bromide, sodium iodide, and potassium iodide. Examples of nitrates include sodium nitrate and potassium nitrate. Examples of acetates include sodium acetate. Examples of carbonates include ferrous carbonate. Examples of metal sulfates include potassium sulfate, sodium sulfate, and ferrous sulfate.

The water retaining agent is not limited so far as it is able to retain water. Examples thereof include porous materials derived from plants having high capillary function and hydrophilicity such as wood meal, pulp powder, active carbon, saw dust, cotton cloth having a number of cotton fluffs, short fiber of cotton, paper dust, and vegetable materials, water-containing magnesium silicate based clay minerals such as active clay and zeolite, pearlite, vermiculite, silica based porous substances, coralline stone, and volcanic ash based substances (for example, terraballoon, *shirasu* balloon, and *taisetsu* balloon). In order to increase a water retaining ability and enhance a shape holding ability of such a water retaining agent, the water retaining agent may be subjected to a processing treatment such as baking and/or pulverization.

The water absorptive polymer is not particularly limited so far as it is a resin having a crosslinking structure and having a water absorption magnification of ion-exchanged water of 3 times or more of the dead weight. Furthermore, a water absorptive polymer the surface of which is crosslinked may be employed. Conventionally known water absorptive polymers and commercial products may also be employed.

Examples of the water absorptive polymer include poly(meth)acrylic acid crosslinked materials, poly(meth)-acrylic acid salt crosslinked materials, sulfonic group-containing poly(meth)acrylic ester crosslinked materials, polyoxyalkylene group-containing poly(meth)acrylic ester crosslinked materials, poly(meth)acrylamide crosslinked materials, crosslinked materials of a copolymer of a (meth)acrylic acid salt and a (meth)acrylamide, crosslinked materials of a copolymer of a hydroxyalkyl (meth) acrylate and a (meth)acrylic acid salt, polydioxolane crosslinked materials, crosslinked polyethylene oxide, crosslinked polyvinylpyrrolidone, sulfonated polystyrene crosslinked materials, crosslinked polyvinylpyridine, saponification products of a starch-poly(meth)acrylonitrile graft copolymer, starch-poly(meth)acrylic acid (salt) graft crosslinked copolymers, reaction products of polyvinyl alcohol and maleic anhydride (salt), crosslinked polyvinyl alcohol sulfonic acid salts, polyvinyl alcohol-acrylic acid graft copolymers, and polyisobutylene maleic acid (salt) crosslinked polymers. These water absorptive polymers may be used alone or in combination with two or more kinds thereof.

Of these water absorptive polymers, water absorptive polymers having biodegradation properties are not limited so far as they are a biodegradable water absorptive polymer. Examples thereof include polyethylene oxide crosslinked materials, polyvinyl alcohol crosslinked materials, carboxymethyl cellulose crosslinked materials, alginic acid crosslinked materials, starch crosslinked materials, polyamino acid crosslinked materials, and polylactic acid crosslinked materials.

The pH adjusting agent is not limited so far it is able to adjust the pH. Examples thereof include alkali metal weak acid salts and hydroxides and alkaline earth metal weak acid salts and hydroxides such as $Na_2CO_3$, $NaHCO_3$, $Na_3PO_4$, $Na_2HPO_4$, $Na_5P_3O_{10}$, NaOH, KOH, $Ca(OH)_2$, $Mg(OH)_2$, and $Ca_3(PO_4)_2$.

The hydrogen formation inhibitor is not limited so far as it is able to inhibit the formation of hydrogen. Examples thereof include one member or two or more members selected from the group consisting of sulfur compounds, oxidizing agents, alkaline substances, sulfur, antimony, selenium, phosphorus, and tellurium. Incidentally, examples of sulfur compounds include compounds with an alkali metal or an alkaline earth metal, metal sulfides such as calcium sulfide, metal sulfites such as sodium sulfite, and metal thiosulfates such as sodium thiosulfate.

Examples of the oxidizing agent include nitrates, oxides, peroxides, halogenated oxygen acid salts, permanganates, and chromates.

The aggregate is not limited so far as it is useful as a filler and/or is useful for making the heat generating composition porous. Examples thereof include fossilized coral (for example, coral fossil and weathered coral fossil), bamboo charcoal, *bincho* charcoal, silica-alumina powders, silica-magnesia powders, kaolin, crystalline cellulose, colloidal silica, pumice, silica gel, silica powders, mica powders, clays, talc, synthetic resin powders or pellets, foamed synthetic resins such as foamed polyesters or polyurethanes, diatomaceous earth, alumina, and cellulose powder. Incidentally, it is to be noted that kaolin and crystalline cellulose are not contained in the heat generating composition of the invention.

The fibrous material is an inorganic fibrous material and/or an organic fibrous material. Examples thereof include rock wool, glass fibers, carbon fibers, metal fibers, pulps, papers, non-woven fabrics, woven fabrics, natural fibers such as cotton and hemp, regenerated fibers such as rayon, semi-synthetic fibers such as acetates, synthetic fibers, and pulverized products thereof.

The functional substance is not limited so far as it is a substance having any function. Examples thereof include at least one member selected from minus ion emitting substances and far infrared ray radiating substances. The minus ion emitting substance is not limited so far as it emits a minus ion as a result either directly or indirectly, and examples thereof include ferroelectric substances such as tourmaline, fossilized coral, granite, and calcium strontium propionate, and ores containing a radioactive substance such as radium and radon. The far infrared ray radiating substance is not limited so far as it radiates far infrared rays. Examples thereof include ceramics, alumina, zeolite, zirconium, and silica.

The surfactant includes anionic surfactants, cationic surfactants, nonionic surfactants, and ampholytic surfactants. Especially, nonionic surfactants are preferable, and examples thereof include polyoxyethylene alkyl ethers, alkylphenol-ethylene oxide adducts, and higher alcohol phosphoric acid esters.

The organosilicon compound is not limited so far as it is a compound having at least an Si-O-R bond and/or an Si-N-R bond and/or an Si-R bond. The organosilicon compound is in the form of a monomer, a lowly condensed product, a polymer, etc. Examples thereof include organosilane compounds such as methyltriethoxysilane; and dimethylsilicone oil, polyorganosiloxane, or silicone resin compositions containing the same.

The pyroelectric substance is not limited so far as it has pyroelectricity. Examples thereof include tourmaline, hemimorphic ores, and pyroelectric ores. Tourmaline or achroite which is a kind of tourmaline is especially preferable. Examples of the tourmaline include dravite, schorl, and elbaite.

The moisturizer is not limited so far as it is able to hold moisture. Examples thereof include hyaluronic acid, collagen, glycerin, and urea.

The fertilizer component is not limited so far as it is a component containing at least one of three elements of nitrogen, phosphorus and potassium. Examples thereof include a bone powder, urea, ammonium sulfate, calcium perphosphate, potassium chloride, and calcium sulfate.

The hydrophobic polymer compound is not limited so far as it is a polymer compound having a contact angle with water of 40° or more, preferably 50° or more, and more preferably 60° or more in order to improve the draining in the composition. The shape of the hydrophobic polymer compound is not limited, and examples thereof include powdery, particulate, granular, and tablet shapes. Examples of the hydrophobic polymer compound include polyolefins such as polyethylene and polypropylene, polyesters, and polyamides.

Examples of the heat generating aid include metal powders, metal salts, and metal oxides such as Cu, Mn, $CuCl_2$, $FeCl_2$, manganese dioxide, cupric oxide, triiron tetroxide, and mixtures thereof.

As the metal oxide other than iron oxide, any material can be employed so far as it does not hinder the oxidation of iron by an oxidizing gas, and examples thereof include manganese dioxide and cupric oxide.

The acidic substance may be any of an inorganic acid, an organic acid, or an acidic salt. Examples thereof include hydrochloric acid, sulfuric acid, nitric acid, acetic acid, oxalic acid, citric acid, malic acid, maleic acid, chloroacetic acid, iron chloride, iron sulfate, iron oxalate, iron citrate, aluminum chloride, ammonium chloride, and hypochlorous acid.

As the "iron powder" as referred to herein, usual iron powders, iron alloy powders and active iron powders such as iron powders comprising particles, a surface of each of which is at least partially covered with an oxygen-containing film , and iron alloy powders comprising particles , a surface of each of which is at least partially covered with an oxygen-containing film, are preferable. Incidentally, the "iron oxide film" as referred to herein is a film made of oxygen-containing iron such as iron oxide, hydroxide or oxyhydroxide. Furthermore, the "active iron powder" as referred to herein is a powder in which an iron oxide film is formed at least locally on the surface of an iron powder, from which an oxidation reaction promoting effect is obtained by a local cell as formed between an iron matrix and an iron oxide film or a pit inside and outside the iron oxide film.

The iron powder is not limited, and examples thereof include cast iron powders, atomized iron powders, electrolyzed iron powders, reduced iron powders, sponge iron powders, and iron alloy powders thereof. In addition, the iron powder may contain carbon or oxygen, and an iron powder containing 50 % or more of iron and other metals may be employed.

The kind of the metal which is contained as an alloy, etc. is not particularly limited so far as the iron component works as a component of the heat generating composition. Examples of such a metal include metals such as aluminum, manganese, copper, nickel, silicon, cobalt, palladium, and molybdenum, and semiconductors. The metal of the invention includes a semiconductor. Such a metal or alloy may be contained only in the surface or the interior, or may be contained in both the surface and the interior.

In the iron powder of the invention, the content of the metal other than iron is usually from 0.01 to 50 % by weight, and preferably from 0.1 to 10 % by weight based on the whole of the iron powder.

Examples of the iron powder having an oxygen-containing film on at least a part of the surface of the iron include:

(A) an active iron powder in which the surface of an iron component is at least partially oxidized, which is obtained by contact treating the essential components of the heat generating composition or the essential components to which acidic substances or other necessary components are added with an oxidizing gas, thereby partially oxidizing the iron component;

(B) an active iron powder in which the content of wustite is from 2 to 50 % by weight in terms of an X-ray peak intensity ratio to iron;

(C) an iron powder having an iron oxide film having a thickness of 3 nm or more on the surface thereof; and

(D) a mixture of an active iron powder and an iron powder other than an active iron powder.

With respect to (A), although the mechanism is not elucidated in detail, it is assumed that upon contact between the oxidizing gas and the components, not only an iron oxide film, namely, an oxygen-containing film is formed on the surface of the iron powder due to the oxidation of the components, especially the oxidation of the iron powder, but also the surface of active carbon is oxidized and/or the oxidized iron component is adhered, whereby hydrophilicity is imparted or improved, and coupling between the components or structurization takes place through the mediation of water.

That is, it is assumed that some kind of a change in the function occurs such that an iron oxide film is formed on the surface of the iron powder, the shape of the iron powder particle becomes irregular, a strain is generated due to the oxidation, or a water-containing pit is formed, whereby the iron powder is activated and exothermic rising properties are improved.

Furthermore, the case where magnetite ($Fe_3O_4$) is present in the iron oxide film is preferable because the conductivity is excellent, and the case where hematite ($Fe_2O_3$) is present in the iron oxide film is also preferable because the iron oxide film becomes porous. Moreover, it is assumed that the carbon component is oxidized on the surface thereof and becomes a carbon component which is rich in oxides on the surface thereof, whereby the hydrophilicity increases and the activity increases.

The thickness of the iron oxide film which is an oxygen-containing film covering the surface of the iron powder, as measured by the Auger electron spectroscopy, is usually 3 nm or more, preferably from 3 nm to 100 $\mu m$, more preferably from 30 nm to 100 $\mu m$, further preferably from 30 nm to 50 $\mu m$, still further preferably from 30 nm to 1 $\mu m$, even further preferably from 30 nm to 500 nm, and even still further preferably from 50 nm to 300 nm.

When the thickness of the oxygen-containing film of iron is 3 nm or more, the thickness of the oxygen-containing film of iron is able to exhibit a promoting effect of the oxidation reaction, and upon contact with an oxidizing gas such as air, is able to immediately initiate the oxidation reaction. When the thickness of the oxygen-containing film of iron is 100 $\mu m$ or more, though the heat generation time may possibly be shortened, such is applicable depending upon the utility.

Furthermore, according to the active iron powder, by using a reaction mixture containing, as essential components, an iron powder, a reaction accelerator and water and having a water content of from 0.5 to 20 % by weight and a water mobility value showing a surplus water content of less than 0.01, the reaction rate at the time of the contact treatment with an oxidizing gas can be raised, thereby achieving a time required for regulating a temperature rise of the reaction mixture at 1 °C or more within 10 minutes. By shortening a time required for arrival at a prescribed temperature or higher, proper activation can be achieved, and unnecessary oxidation on the iron powder can be prevented.
Furthermore, the heat generating composition prepared by adding a carbon component, etc. to a heat generating mixture as produced by contact treating the reaction mixture with an oxidizing gas or adjusting the water content so as to have a water mobility value of from 0.01 to 50 is properly tacky, has excellent moldability and is able to be applied with a molding method such as a force-through die molding method and a cast molding method, whereby heat generating bodies of various shapes can be produced. In particular, a heat generating composition having a water mobility value of from 0.01 to 20 is excellent because it initiates an exothermic reaction immediately after contacting with air, has excellent exothermic rising properties and has excellent moldability.
The contact treatment method of the reaction mixture with an oxidizing gas is not particularly limited so far as it is able to contact treat a reaction mixture containing, as essential components, an iron powder, a reaction accelerator and water and having a water content of from 0.5 to 20 % by weight and a water mobility value of less than 0.01 with an oxidizing gas and regulate a temperature rise of the reaction mixture at 1 °C or more.
Specific examples thereof include:

(1) a process for producing a heat generating mixture containing an iron powder having an iron oxide film on the surface thereof by subjecting a reaction mixture of an iron powder, a reaction accelerator and water in an oxidizing gas atmosphere to a self-exothermic reaction, thereby partially oxidizing the iron powder;
(2) a process for producing a heat generating mixture by subjecting a reaction mixture of an iron powder, a reaction accelerator, an acidic substance and water in an oxidizing gas atmosphere to a self-exothermic reaction;
(3) a process for producing a heat generating mixture by subjecting a reaction mixture of an iron powder, a reaction accelerator, a carbon component and water in an oxidizing gas atmosphere to a self-exothermic reaction;
(4) a process for producing a heat generating mixture by subjecting a reaction mixture of an iron powder, a reaction accelerator, an acidic substance, a carbon component and water in an oxidizing gas atmosphere to a self-exothermic reaction;
(5) a process for producing a heat generating mixture containing a partially oxidized iron powder by carrying out the method as set forth above in any one of (1) to (4), wherein the reaction mixture or heat generating mixture as set forth above in any one of (1) to (4) contains a component other than the foregoing components;
(6) a process for producing a heat generating mixture by carrying out the method as set forth above in any one of (1) to (5) under circumstances heated so as to have temperature of at least 10 °C higher than the circumferential temperature;
(7) a process for producing a heat generating mixture by carrying out the method as set forth above in any one of (1) to (6) by blowing an oxidizing gas;
(8) a process for producing a heat generating mixture by carrying out the method as set forth above in (7) by blowing the oxidizing gas heated so as to have a temperature of at least 10 °C higher than the circumferential temperature;
(9) a process for producing a heat generating composition by carrying out the method as set forth above in any one of (1) to (8) by contact treating with an oxidizing gas until the temperature exceeds a maximum temperature which is a maximum point of temperature rise by the exothermic reaction;
(10) a process for producing a heat generating mixture by carrying out the method as set forth above in any one of (1) to (8) by contact treating with an oxidizing gas until the temperature exceeds a maximum temperature by the exothermic reaction and drops by at least 10 to 20 °C from the maximum temperature;
(11) a process for producing a heat generating composition by carrying out the method as set forth above in any one of (1) to (8) by contact treating with an oxidizing gas until the temperature exceeds a maximum temperature which is a maximum point of temperature rise by the exothermic reaction and after intercepting the oxidizing gas, holding it until the temperature of at least the reaction mixture drops by at least 10 to 20 °C from the maximum temperature; and
(12) a process for producing a heat generating mixture by heating the reaction mixture or heat generating mixture as set forth above in any one of (1) to (5) under oxidizing gas circumstances while regulating a temperature rise at 1 °C or more.

In addition, a heat generating mixture as prepared by adding other components to the heat generating mixture and further treating with an oxidizing gas may be employed.
Incidentally, the circumstances of the reaction mixture at the time of contact treatment with an oxidizing gas are not limited so far as the reaction mixture is brought into contact with an oxidizing gas under circumstances of 0 °C or higher and a temperature rise of the reaction mixture is regulated at 1 °C or more within 10 minutes. In the case where the contact treatment is carried out in an open system, the circumstances may be either the state that the reaction mixture is present in a lid-free vessel or the state that an oxidizing gas such as air comes into a vessel through an air-permeable sheet-like material such as non-woven fabrics.

Furthermore, the contact treatment with an oxidizing gas may be carried out with or without stirring in a fluidized or non-fluidized state and may be carried out in a batch or continuous system.

Examples of the final heat generating composition include:

1) a heat generating composition containing, as a heat generating composition raw material, a heat generating mixture produced in the process as set forth above in any one of (1) to (12);
2) a heat generating composition obtained by adding other components to the heat generating composition as set forth above in 1); and
3) a heat generating composition obtained by adjusting the water content of the heat generating composition as set forth above in 1) or 2).

The order of the timing of adding other components than the essential components and the timing of adjusting the water content is not limited.

Here, the water content in the reaction mixture and also the heat generating mixture prior to the treatment with an oxidizing gas is usually from 0.5 to 20 % by weight, preferably from 1 to 15 % by weight, more preferably from 2 to 10 % by weight, further preferably from 3 to 10 % by weight, and still further preferably from 6 to 10 % by weight.

The temperature of the reaction mixture after the contact with an oxidizing gas is not limited so far as the temperature rise is regulated at 1 °C or more. The temperature of the reaction mixture after the contact with an oxidizing gas is preferably from 1 to 80 °C, more preferably from 1 to 70 °C, further preferably from 1 to 60 °C, and still further preferably from 1 to 40 °C.

The circumferential temperature at the time of contact between the reaction mixture and the oxidizing gas is not limited so far as the temperature of the reaction mixture is raised to a prescribed temperature or higher. The circumferential temperature at the time of contact between the reaction mixture and the oxidizing gas is preferably 0 °C or higher, more preferably from 0 to 250 °C, further preferably from 10 to 200 °C, still further preferably from 20 to 150 °C, even further preferably from 25 to 100 °C, and even still further preferably from 25 to 50 °C.

The time of contact between the reaction mixture and the oxidizing gas is not limited so far as the time required for regulating a temperature rise at 1 °C or more is within 10 minutes. The time of contact between the reaction mixture and the oxidizing gas is preferably from one second to 10 minutes, more preferably from one second to 7 minutes, further preferably from one second to 5 minutes, still further preferably from 2 seconds to 5 minutes, even further preferably from 2 seconds to 3 minutes, and even still further preferably from 2 seconds to one minute.

The temperature of the oxidizing gas is not limited so far as the foregoing circumferential temperature is kept.

As the "oxidizing gas" as referred to herein, any gas can be used as the oxidizing gas so far as it is oxidizing. Examples thereof include an oxygen gas, air, and mixed gases of an inert gas (for example, a nitrogen gas, an argon gas, and a helium gas) and an oxygen gas. Although the mixed gas is not limited so far as it contains oxygen, mixed gases containing 10 % or more of an oxygen gas are preferable, and of these, air is especially preferable. If desired, a catalyst such as platinum, palladium, iridium, and compounds thereof can also be used.

The oxidation reaction can be carried out under stirring in an oxidizing gas atmosphere optionally under a pressure and/or upon irradiation of ultrasonic waves.

The optimal condition of the oxidation reaction may be properly experimentally determined.

An amount of the oxidizing gas to be used is not limited but may be adjusted depending upon the kind of the oxidizing gas, the kind and particle size of the iron powder, the water content, the treatment temperature, the treatment method, and the like.

In the case of an open system, there is no limitation so far as a necessary amount of oxygen can be taken in. In order to prevent fly of the reaction mixture or contamination of dusts, etc., the system may be surrounded by an air-permeable raw material such as non-woven fabrics and woven fabrics. So far as the system is in an air-permeable state, it is to be noted that the system is an open system.

In the case where air is used in the system of blowing an oxidizing gas, for example, the amount of air is preferably from 0.01 to 1,000 L/min, more preferably from 0.01 to 100 L/min, and further preferably from 0.1 to 50 L/min per 200 g of the iron powder under one atmosphere. In the case of other oxidizing gas, the amount of the oxidizing gas may be converted on the basis of the case of air.

If desired, a peroxide may be added. Examples of the peroxide include hydrogen peroxide and ozone.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of the contact treatment with a mixed oxidizing gas at the time of adjusting the water content are not limited, and examples thereof include those in an oxidizing gas atmosphere and those in blowing of an oxidizing gas.

A method for measuring a temperature rise of the heat generating composition is as follows.

1) A heat generating composition is allowed to stand in a state that it is sealed in an air-impermeable outer bag for one hour under a condition that the circumferential temperature is 20 ± 1 °C.

2) A magnet is provided in the vicinity of a central part of the back side of a polyvinyl chloride-made supporting plate (3 mm in thickness × 600 mm in length × 600 mm in width) of a footed supporting table so as to cover a cavity shape of a molding die.

3) A temperature sensor is placed on the central part of the supporting plate.

4) A polyethylene film (25 $\mu$m in thickness × 250 mm in length × 200 mm in width) as provided with an adhesive layer having a thickness of about 80 $\mu$m is stuck onto the supporting plate via a sticky layer such that the center of the polyethylene film is positioned at the sensor.

5) The heat generating composition is taken out from the outer bag.

6) A template (250 mm in length × 200 mm in width) having a cavity (80 mm in length × 50 mm in width × 3 mm in height) is placed above the central part of the polyethylene film; a sample is placed in the vicinity of the cavity; a force-in die plate is moved along the template; the sample is charged into the cavity while stuffing; and the sample is leveled while stuffing along the template plane (force-in die molding), thereby filling the sample in the die. Next, the magnet beneath the supporting plate is removed, and the temperature measurement is started.

With respect to the measurement of the exothermic temperature, the temperature is measured for 10 minutes at a measurement timing of 2 seconds using a data collector, and exothermic rising properties are judged in terms of the temperature after elapsing 3 minutes.

The heat generation test of the heat generating body follows the JIS temperature characteristic test.

In the iron powder or active iron powder in the oxidizing gas-treated heat generating composition, at least a part of the surface thereof is covered by an oxygen-containing film of iron. The degree of covering on the surface of the oxygen-containing film of iron is not limited so far as at least a part of the surface thereof is covered, and the surface may be entirely covered. In the case of the heat generating composition of the invention, since an ion of the reaction accelerator such as a chlorine ion is contained in the heat generating composition, there is no corrosion effect of the oxide film due to anti-corrosion effect by the ion of the reaction accelerator such as a chlorine ion. Thus, the oxidation reaction which is a sort of corrosion is not hindered. In particular, in the case where an oxygen-containing film of iron is prepared while the ion of the reaction accelerator such as a chlorine ion exists together, the subject effect is large. In the case where a metal other than iron is present on the surface, it is only required that at least other part of the metal portion other than iron is covered by the oxygen-containing film of iron.

In the iron powder of the invention, not only a region where (1) entire (uniform) corrosion, (2) pitting or crevice corrosion, (3) stress corrosion cracking, or the like is generated, but also irregularities or crevices are formed.

For that reason, it is assumed that the iron powder of the invention has hydrophilicity and oxidation catalytic properties (FeO, etc.) in its own portion. In producing the heat generating composition, it is important that the iron powder has an oxygen-containing film in its own portion without relying upon mixing. In particular, in the iron component as prepared by contact treating the iron component and the reaction accelerator and water as essential components with an oxidizing gas, it is thought that a reaction active part composed mainly of an oxide, a hydroxide, a chlorine ion, a hydrogen ion, etc. is formed, whereby exothermic reactivity and hydrophilicity are improved and exothermic rising properties and moldability are remarkably improved.

With respect to (B), the amount of FeO (wustite) which is contained in the iron component containing a prescribed amount of wustite is usually from 2 to 50 % by weight, preferably from 2 to 40 % by weight, more preferably from 2 to 30 % by weight, further preferably from 5 to 30 % by weight, and still further preferably from 6 to 30 % by weight in terms of an X-ray peak intensity ratio of iron. When the amount of FeO (wustite) exceeds 50 % by weight, though the exothermic rising properties are good, the duration of heat generation becomes short. On the other hand, when it is less than 2 % by weight, the exothermic rising properties become dull.

The thickness of the oxygen-containing film of a prescribed amount or the oxygen-containing film of iron powder containing wustite and the amount of wustite are applied to the heat generating composition or the heat generating composition molded body at the time of lamination.

An iron powder containing a carbon component and/or covered by a carbon component is also preferable. Although a proportion of the carbon component is not limited so far as a ratio of the iron component to the carbon component is 50 % by weight or more, an iron powder in which the surface thereof is partially covered by from 0.3 to 3.0 % by weight of a conductive carbonaceous substance is useful. Examples of the conductive carbonaceous substance include carbon black, active carbon, carbon nanotubes, carbon nanohorns, and flullerenes. Ones which have become conductive by doping are also employable. Examples of the iron powder include reduced iron powders, atomized iron powders, and sponge iron powders. In particular, the case where the conductive carbonaceous substance is active carbon and the iron powder is a reduced iron powder is useful as a heat generating body.

Furthermore, in order to efficiently carry out covering by a conductive carbonaceous substance, an oil such as a spindle oil may be added in an amount of from 0.01 to 0.05 % by weight to such an extent that the fluidity of the iron powder is not hindered.

In the case of measuring the water mobility value of the heat generating composition in the heat generating body and the thickness and amount of wustite of the iron oxide film of iron powder in the mixture or the heat generating composition in the heat generating body, the heat generating composition or mixture may be measured according to the following items.

1) Water mobility value:

The heat generating composition is taken out from the heat generating body and measured according to the foregoing method of measuring a water mobility value.

2) Thickness and amount of wustite of iron oxide film of iron powder:

A measuring sample as prepared by dispersing the heat generating composition, the heat generating composition molded body, the heat generating composition compression molded body or the mixture in nitrogen-purged ion-exchanged water in a nitrogen atmosphere, separating the iron powder using a magnet and drying the iron powder in a nitrogen atmosphere is used.

The heat generating composition of the invention contains, as essential components, an iron powder, a carbon component, a reaction accelerator and water, and its production process is one which can be put into practical use on an industrial scale. A reaction mixture containing, as essential components, an iron powder, a reaction accelerator and water and having a water content of from 1 to 20 % by weight and a water mobility value showing a surplus water content of less than 0.01 is brought into contact with an oxidizing gas under circumstances at 0 °C or higher, a temperature rise of the reaction mixture is regulated at 1 °C or more within 10 minutes to produce a heat generating mixture, and the subject heat generating mixture is used as a raw material to form a heat generating composition. Alternatively, a heat generating composition may be formed by subsequently further adjusting the water content, or by further adding a carbon component, etc. and adjusting the water content.

In the invention, it has become possible to realize the contact treatment with an oxidizing gas within a short period of time by regulating the water content of the reaction mixture at a fixed amount or less, especially regulating the surplus water content of the reaction mixture at a fixed amount or less and carrying out an oxidizing contact treatment. By specifying the surplus water content and performing the treatment within a short period of time, adverse influences such as poor initial exothermic rising of the heat generating composition and shortening of the heat generation-retaining time can be avoided. Thus, it has become possible to establish an industrial mass-production process. Furthermore, although stirring or the like may not be achieved during the contact treatment with an oxidizing gas, when stirring or the like is achieved, the contact treatment with an oxidizing gas can be surely carried out.

Here, so far as the iron powder is partially oxidized, the state of the reaction mixture or heat generating mixture at the time of the contact treatment with an oxidizing gas may be any of a standing state, a transfer state, or a fluidizing state by stirring, etc. and may be properly selected. Furthermore, the circumstances at the time of mixing the respective components of the reaction mixture, the heat generating mixture or the heat generating composition and at the time of mixing at the time of adjusting the water content are not limited, and examples thereof include those in an oxidizing gas atmosphere and those in blowing of an oxidizing gas.

The "adjustment of the water content" as referred to herein means that after contact treating the heat generating mixture with an oxidizing gas, water or an aqueous solution of a reaction accelerator is added. Although the amount of addition of water or an aqueous solution of a reaction accelerator is not limited, examples thereof include the addition of a weight corresponding to a reduced weight by the contact treatment and the addition of a weight such that a desired water mobility value is obtained.

Whether or nor the adjustment of the water content is introduced may be properly determined depending upon the utility.

The heat generating composition of the invention contains, as essential components, an iron powder, a carbon component, a reaction accelerator and water and is started from a mixture obtained by contact treating a reaction mixture containing, as essential components, an iron powder, a reaction accelerator and water with an oxidizing gas. The heat generating composition of the invention is usually one obtained by adjusting the water content of a heat generating mixture and is a heat generating composition which is satisfactory in the exothermic rising, has a suitable amount of surplus water and has excellent moldability. Furthermore, it is possible to produce a heat generating body which can become promptly warm at the time of use.

Accordingly, at least the iron powder further including the carbon component has a history of oxidation by the contact treatment with an oxidizing gas, and it is thought that this is deeply related to excellent exothermic rising properties, exothermic endurance and excellent moldability.

When the iron powder which is contact treated with an oxidizing gas according to the invention is used, the amount of addition of the carbon component (for example, active carbon) in the heat generating composition can be reduced by, for example, 20 % or more. By reducing the amount of addition of the carbon component, the costs are lowered.

According to the production process of the heat generating mixture of the invention, it is possible to obtain a heat generating composition having excellent exothermic rising properties, excellent hydrophilicity, and excellent moldability. In particular, a heat generating composition having remarkably excellent moldability and exothermic characteristics together can be obtained while specifying the water availability value at from 0.01 to 50, in particular 0.01 to 20.

The heat generating composition as produced by the production process of the invention is remarkably improved with respect to exothermic rising properties. Thus, the amount of addition of the carbon component (such as active carbon) in the heat generating composition can be reduced by, for example, 20 % or more so that it can contribute to a reduction in costs.

Furthermore, since the hydrophilicity is remarkably improved, the moldability with a mold is remarkably improved. Thus, since after molding, collapsed pieces of the heat generating composition are not scattered on the surroundings of the heat generating composition molded body, sealing can be appropriately achieved so that a heat generating body free from sealing cut can be produced. In this way, heat generating composition molded bodies of various shapes can be produced, and heat generating bodies of various shapes are formed.

Furthermore, in view of improving the exothermic rising properties of the heat generating composition, the following are preferable.

1) A heat generating composition obtained by a contact treatment (self heat generation) of a mixture of the essential components of the heat generating composition, or a mixture of the foregoing mixture and an acidic substance or other necessary components with an oxidizing gas, a heat generating composition obtained by additionally adjusting the water content of the foregoing heat generating composition, or a heat generating composition obtained by adding and mixing other components in the foregoing heat generating composition.

2) Any one of the following active iron powders having an oxygen-containing film (for example, oxides) on at least a part of the surface thereof is used as the iron powder: (a) an iron powder having an oxygen-containing film of iron having a thickness, as measured by the Auger electron spectroscopy, of 3 nm or more on the surface thereof and (b) an iron powder having a content of wustite of from 2 to 50 % by weight in terms of an X-ray peak intensity ratio to iron.

3) A mixture of an active iron powder having an oxygen-containing film (for example, oxides) on at least a part of the surface thereof and an iron powder not having an oxygen-containing film is used as the iron powder. In this case, a mixture containing 60 % by weight or more of an active iron powder and less than 40 % by weight of an iron powder other than the active iron is preferable.

In the case of storing the heat generating composition which is treated with an oxidizing gas or the heat generating composition containing an active iron powder, or a material utilizing the same over a long period of time, it is preferred to combine a hydrogen formation inhibitor therewith. This is because in this way, a heat generating body having excellent exothermic characteristics, which is inhibited in the formation of hydrogen, is free from swelling of the outer bag at the time of storage, etc. and has satisfactory exothermic rising properties, is obtained.

Furthermore, so far as the rising characteristics are not affected, the heat generating composition having a water mobility value falling outside the range of from 0.01 to 20 can contain a water-soluble polymer, a flocculant aid, a flocculant, an agglomeration aid, a dry binding material, a dry binding agent, a dry binder, an adhesive raw material, a tackifier, an excipient, a flocculating agent, or a soluble sticky raw material.

Furthermore, since a marketed heat generating body in which a heat generating composition is accommodated in an accommodating bag is provided on the assumption that it is accommodated in an outer bag which is an air-impermeable accommodating bag and is storable over a long period of time, it is preferred to use a heat generating composition containing a hydrogen formation inhibitor. Since the heat generating composition which has passed through the contact treatment with an oxidizing gas is an active composition, it is important that the heat generating composition contains a hydrogen formation inhibitor. Also, this efficacy is further strengthened by using a pH adjusting agent together.

Furthermore, so far as the reaction characteristics and exothermic characteristics are not affected, the heat generating composition having a water mobility value of less than 0.01 may contain a flocculant aid, a flocculant, an agglomeration aid, a dry binder, a dry binding agent, a dry binding material, a sticky raw material, a thickener, an excipient, or a water-soluble polymer in an amount ranging from 0.01 to 3 parts by weight respectively.

The "flocculant aid" as referred to herein is a flocculant aid as described in Japanese Patent No. 3,161,605 (JP-T-11-508314) such as gelatin, natural gum, and corn syrup.

The "flocculant" as referred to herein is a flocculant as described in JP-T-2002-514104 such as corn syrup and maltitol syrup.

The "agglomeration aid" as referred to herein is an agglomeration aid as described in JP-T-2001-507593 such as corn syrup.

The "dry binder" as referred to herein is a dry binder as described in JP-T-2002-514104 such as microcrystalline cellulose, maltodextrin, and mixtures thereof.

The "dry binding agent" as referred to herein is a dry binding agent as described in JP-T-2001-507593 such as maltodextrin and sprayed lactose.

The "dry binding material" as referred to herein is a dry binding material as described in JP-T-11-508314 such as microcrystalline cellulose, maltodextrin, and mixtures thereof.

The "sticky raw material" or the "binder" as referred to herein is a sticky raw material or binder as described in JP-A-4-293989 such as water glass, polyvinyl alcohol (PVA), and carboxymethyl cellulose (CMC).

The "thickener" as referred to herein is a thickener as described in JP-A-6-343658 such as corn starch and potato starch.

The "excipient" as referred to herein is an excipient as described in JP-A-7-194641 such as $\alpha$-starch and sodium alginate.

As the "water-soluble polymer" as referred to herein, the water-soluble polymer in the adhesive layer can be used.

The particle size of the water-insoluble solid component constituting the moldable heat generating composition of the invention is not limited so far as the heat generating composition has moldability. In the case where any one of length, width and height as the size of the heat generating composition molded body as molded from the heat generating composition is small, the moldability is improved by making the particle size small.

In addition, it is preferable in view of molding that the particle size of the solid component constituting the moldable heat generating composition is small. A maximum particle size of the water-insoluble solid component exclusive of the reaction accelerator and water in the components constituting the moldable heat generating composition is preferably not more than 2.5 mm, more preferably not more than 930 $\mu$m, further preferably not more than 500 $\mu$m, still further preferably not more than 300 $\mu$m, even further preferably not more than 250 $\mu$m, and even still further preferably not more than 200 $\mu$m. Moreover, 80 % or more of the particle size of the solid component is usually not more than 500 $\mu$m, preferably not more than 300 $\mu$m, more preferably not more than 250 $\mu$m, further preferably not more than 200 $\mu$m, still further preferably not more than 150 $\mu$m, and even further preferably not more than 100 $\mu$m.

Incidentally, with respect to the particle size of the water-insoluble solid component, separation is conducted using a sieve, and the particle size of the component which has passed through the sieve is calculated from an opening of the sieve. That is, sieves of 8, 12, 20, 32, 42, 60, 80, 100, 115, 150, 200, 250 and 280 meshes and a receiving dish are combined in this order from up to down. About 50 g of water-insoluble solid component particles are placed on the uppermost 8-mesh sieve and shaken for one minute using an automatic shaker. Weights of the water-insoluble solid component particles on each of the sieves and the receiving dish are weighed. The total amount thereof is defined as 100 %, and the particle size distribution is determined from weight fractions. When the sum of all receiving dishes under the sieve of a specific mesh size becomes 100 % which is the total sum of the particle size distribution, the size ($\mu$m) calculated from the opening of the specific mesh is defined as the particle size of the water-insoluble solid component. Incidentally, each of the mesh sieves may be combined with other mesh sieves. Here, the particles which have passed through a 16-mesh sieve are defined to have a particle size of not more than 1 mm; the particles which have passed through a 20-mesh sieve are defined to have a particle size of not more than 850 $\mu$m; the particles which have passed through a 48-mesh sieve are defined to have a particle size of not more than 300 $\mu$m; the particles which have passed through a 60-mesh sieve are defined to have a particle size of not more than 250 $\mu$m; the particles which have passed through a 65-mesh sieve are defined to have a particle size of not more than 200 $\mu$m; the particles which have passed through an 80-mesh sieve are defined to have a particle size of not more than 180 $\mu$m; the particles which have passed through a 100-mesh sieve are defined to have a particle size of not more than 150 $\mu$m; the particles which have passed through a 115-mesh sieve are defined to have a particle size of not more than 120 $\mu$m; the particles which have passed through a 150-mesh sieve are defined to have a particle size of not more than 100 $\mu$m; and the particles which have passed through a 250-mesh sieve are defined to have a particle size of not more 63 $\mu$m, respectively. The same is applicable to mesh sizes of less than these mesh sizes.

Furthermore, the heat generating composition can be classified into a powder, a granulate heat generating composition (having a water mobility value of less than 0.01), a moldable heat generating composition (having a water mobility value of from 0.01 to 20), and a sherbet-like heat generating composition (having a water mobility value exceeding 20 but not more than 50) depending upon the state of adjustment of the water content or surplus water. The heat generating composition as classified depending upon the water mobility value is as described previously.

The process for producing a microheater of the invention is not limited so far as it is a process for producing a microheater by forming the shape by using a magnetic or a die and molding the moldable heat generating composition into a desired shape. Examples thereof include a force-through molding method and a cast molding method. That is, a heat generating composition molded body resulting from molding a moldable heat generating composition which contains, as essential components, an iron powder, a carbon component, a reaction accelerator and water, does not contain a flocculant aid, a dry binding agent and a flocculant and contains surplus water so as to have a water mobility value of from 0.01 to 20 by force-through molding or the like is laminated on a substrate which is substantially planar and does not have an accommodating pocket, the heat generating composition molded body is then covered by a covering material, and the surroundings of the heat generating composition molded body are heat sealed, thereby forming a microheater.

It is also possible to prepare a microheater by carrying out molding by cast molding in place of the force-through molding and similarly laminating the heat generating composition molded body on a substrate which is substantially planar and does not have an accommodating pocket.

Furthermore, the moldable heat generating composition or the heat generating composition molded body may be compressed. The compression method is not limited, and examples thereof include a method for subjecting the moldable heat generating composition or the moldable heat generating composition molded body to in-die compression or out-die compression. It is assumed that when a pressure is applied to the moldable heat generating composition having surplus water having a water mobility value of from 0.01 to 20, particles are brought into contact with each other, whereby the particles are fixed by a frictional force or a surface tension of surplus water and become in a sand dumpling state. This is laminated on a substrate, covered by a covering material and then sealed, thereby revealing shape holding properties sufficient for processing into a microheater. It is to be noted that the compressed body of the heat generating composition or heat generating composition molded body is defined as a heat generating composition compressed body and included in the heat generating composition molded body.

The "in-die compression" as referred to herein means that the heat generating composition is compressed by using a flexible rubber roll or the like and pushing a rubber roll or the like into a cavity while being deformed or pushing a force-in die having a force-in shape adaptive to the shape of a cavity or the like, during a time when the moldable heat generating composition is present within the die. This method includes subsequent lamination of the compressed moldable heat generating composition molded body on a substrate which is substantially planar and does not contain an accommodating pocket. On the other hand, the "out-die compression" as referred to herein means that after the moldable heat generating composition leaves from a die and is laminated as a moldable heat generating composition molded body on a substrate, the moldable heat generating composition molded body is compressed by a roll or the like. Though this compression is usually carried out after covering the moldable heat generating composition molded body by an underlay material and/or a covering material, it may be carried out prior to covering.

Furthermore, the heat sealing may be carried out after providing an adhesive such as hot melt based adhesives on at least the surroundings of a substrate on which the heat generating composition laminate has been laminated, covering a covering material thereon and contact bonding to achieve temporary adhesion between the substrate and the covering material. This is useful in the case of high-speed heat sealing.

Here, with respect to the shape, material, raw material and the like of the moldable heat generating composition, substrate, heat generating composition molded body, exothermic part and microheater, all of the foregoing descriptions regarding the microheater can be employed and applied.

The microheater of the invention is produced through a cutting step and so on after the foregoing sealing step. With respect to the sealing step, the cutting step and so on, conventional methods and devices may be properly selected and used.

Furthermore, in the sealing step, sealing is not limited so far as sealing can be achieved. Usually, heat sealing or contact bond sealing or a mixture thereof is employed. With respect to the surface of the seal part, any of a plain surface, a pattern in which the cross-section shape thereof is irregular, or a mixture of a plain surface and a pattern in which the cross-section shape thereof is irregular is employable. The "mixture with a pattern" as referred to herein means that the inside of the seal part is plain and the outside is patterned; that the inside of the seal part is patterned and the outside is plain; or that the seal part is partially plain whereas it is partially patterned. Furthermore, the back side may be plain with the front side being patterned, and vice versa. Furthermore, a part or the whole of the pattern may be a multiple pattern. Accordingly, with respect to the seal roll, a plain or patterned roll is used pursuant thereto. Furthermore, the sealing may be carried out by using a pair of seal rolls or subjected to multiple sealing by using two or more plural pairs of seal rolls. For example, the multiple sealing is double, triple, quadruplet, quintuplet, etc. The seal width may be the same or different and may be properly determined. It is preferable that when the seal speed is high, the number of series is increased. In the case of using heat seal rolls or contact bond seal rolls to which the temperature is applied, the temperature of the pair of rolls may be the same, or the temperature of one roll may be different from that of the other roll. Furthermore, after sealing, the exothermic part may be made flatter by lightly pressing the exothermic part by a roll or the like.

The "force-through molding method" as referred to herein means a molding method in which a trimming die is used, one surface of the cavity of the trimming die is plugged by a belt, a roll, etc. and the heat generating composition is accommodated from the other surface, thereby molding a prescribed shape. For example, by using a molding machine in a form of a body of rotation for laminating a heat generating composition molded body in a trimming die shape on a longitudinal substrate, it is covered by a longitudinal covering material, and the substrate and the covering material are sealed (by heat sealing, contact bonding sealing, or heat contact bonding sealing) in the surroundings of the heart generating composition molded body. Here, if a rotary seal unit is used as a seal unit and the surroundings of the heat generating composition molded body are heat sealed via the seal unit, followed by a sealing treatment, this method becomes a continuous molding method.

The "cast molding method" as referred to herein means a molding method for laminating the heat generating composition molded body on a longitudinal substrate or the like by filling into a casting die having a concave and movement onto the substrate. In the continuous case, by using a molding machine for laminating a heat generating composition molded body on a longitudinal substrate by filling in a concave and movement onto a substrate by a rotatory body of rotation, it is covered by a longitudinal covering material, and the desired sectioned part, the substrate and the surroundings of the covering material are sealed (by heat sealing, contact bonding sealing, or heat contact bonding sealing). Here, if a rotary seal unit is used as a seal unit and the surroundings of the heat generating composition molded body are heat sealed via the seal unit, followed by a sealing treatment, this method becomes a continuous molding method.

Furthermore, a magnet may be used for molding the moldable heat generating composition of the invention. The heat generating composition may be formed into a desired shape by using a magnet as processed into a desired shape. By using a magnet in other methods, it becomes possible to easily achieve accommodation of the moldable heat generating composition in a mold and separation of the molded body from the mold, thereby making it easier to mold a heat generating composition molded body.

Incidentally, the microheater may be produced by providing an air-permeable adhesive layer at least between the heat generating composition molded body and the covering material or providing an underlay material such as non-woven fabrics between the heat generating composition molded body and the covering material.

Furthermore, in the case where an air-permeable sticky layer as constituted of an adhesive layer is provided at least between the heat generating composition molded body and the covering material, there is no limitation so far as an air-permeable sticky layer is present at least between the heat generating composition molded body and the covering material. For example, the air-permeable sticky layer may be provided on the surface of the covering material opposing to the heat generating composition molded body; and the air-permeable sticky layer may be provided on the heat generating composition molded body or on a laminate of the heat generating composition molded body and the substrate and temporarily adhered under pressure or the like between the covering material and the heat generating composition molded body and/or the substrate.

Furthermore, it becomes possible to realize a high-speed production process of the microheater more surely by temporarily adhering the substrate and the heat generating composition molded body as laminated on the substrate and the covering material by contact bonding sealing with a sticky layer and heat sealing the periphery of the heat generating composition molded body.

Though the adhesive of the sticky layer which is used for temporary adhesion is not limited, as one example thereof, the adhesive which constitutes the adhesive layer to be used in the invention is enumerated. The method and form for providing it are not limited. There is no limitation so far as the adhesive layer can secure air permeability by a printing method such as gravure printing, a coating method, a melt flow blow method, a curtain spray method, etc. Examples thereof include a point form, a cobweb form, and a netlike form. In particular, it is preferred to provide the hot melt based adhesive in a cobweb form by a melt blow method, a curtain spray method, etc.

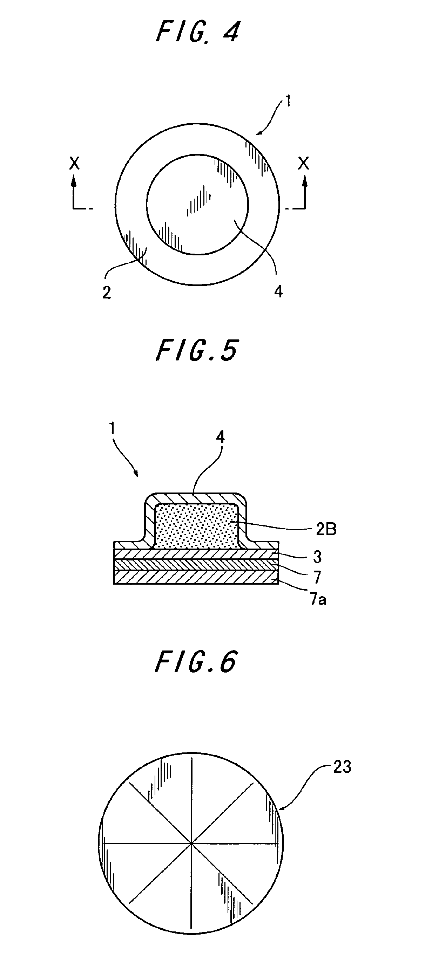

The "water mobility value" as referred to herein is a value showing an amount of surplus water which can transfer to the outside of the heat generating composition in water present in the heat generating composition. This water mobility value will be described below with reference to Figs. 6 to 10.

As shown in Fig. 6, a filter paper 23 of No. 2 (second class of JIS P3801) in which eight lines are drawn radiating from the central point with an interval of 45° is placed on a stainless steel plate 27 as shown in Figs. 7 and 8; a template 24 having a size of 150 mm in length × 100 mm in width and having a hollow cylindrical hole 25 having a size of 20 mm in inner diameter × 8 mm in height is placed in the center of the filter paper 23; a sample 26 is placed in the vicinity of the hollow cylindrical hole 25; and a stuffer plate 24 is moved on and along the template 24 and inserted into the hollow cylindrical hole 25 while stuffing the sample 26, thereby leveling the sample (force-in die molding).

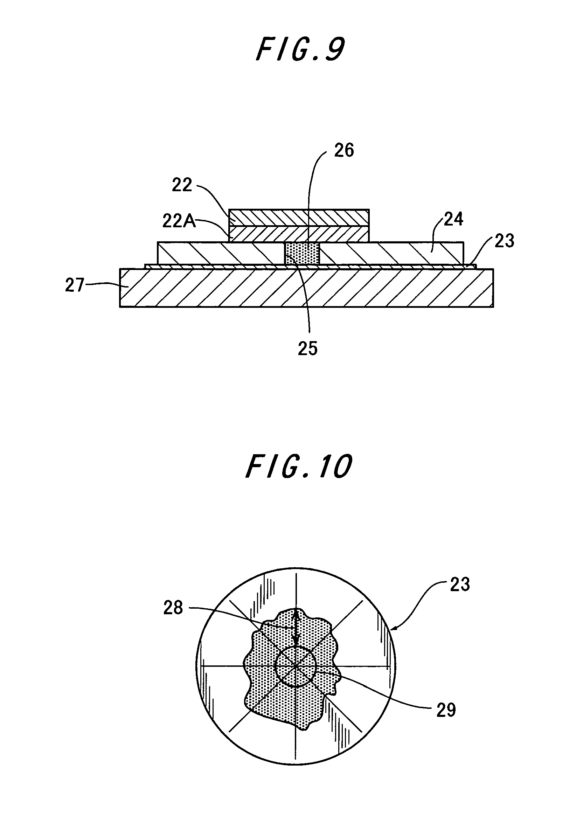

Next, as shown in Fig. 9, a non-water absorptive 70 μm-thick polyethylene film 22A is placed so as to cover the hole 25, and a flat plate 22 made of stainless steel having a size of 5 mm in thickness × 150 mm in length × 150 mm in width is further placed thereon and held for 5 minutes such that an exothermic reaction is not caused.

Thereafter, a shown in Fig. 10, the filter paper 23 is taken out, and an oozed-out locus of the water or aqueous solution is read as a distance 28 (unit: mm) from a periphery 29 as an edge of the hollow cylindrical hole to an oozed-out tip along the radiating lines. Similarly, a distance 28 from each of the lines is read, and eight values in total are obtained. Each of the eight values (a, b, c, d, e, f, g and h) which are read out is defined as a measured water content value. An arithmetic average value of the eight measured water content values is defined as a water content value (mm) of the sample.

Furthermore, the water content for the purpose of measuring a real water content value is defined as a compounded water content of the heat generating composition corresponding to the weight of the heat generating composition having a size of 20 mm in inner diameter × 8 mm in height or the like, similar measurement is conducted only with water corresponding to that water content, and a value as calculated in the same manner is defined as a real water content value (mm). A value obtained by dividing the water content value by the real water content value and then multiplying with 100 is a water mobility value.

That is, the water mobility value is represented by the following expression.

```
(Water mobility value) = {[Water content value (mm)]/

[(Real water content value (mm))] × 100
```

With respect to the same sample, five points are measured, and the five water mobility values are averaged, thereby defining an average value thereof as a water mobility value of the sample.

In the invention, a heat generating body can be formed only by laminating a heat generating composition molded body obtained by molding a heat generating composition having surplus water with a water mobility value of from 0.01 to 20 on a substrate, covering a covering material thereon, and sealing at least the periphery of the heat generating composition molded body. After accommodating it in a packaging material such as a substrate and a covering material, it is not necessary to add water. Accordingly, since the process is remarkably simplified, the invention is superior in view of the costs.

In the invention, the water mobility value (0 to 100) is preferably from 0.01 to 20, more preferably from 0.01 to 18, further preferably from 0.01 to 15, still further preferably from 0.01 to 13, even further preferably from 1 to 13, and even still further preferably from 3 to 13.

In a heat generating body using a heat generating composition molded body obtained by molding a moldable heat generating composition containing surplus water as a connecting substance according to the invention, the heat generating composition contains an appropriate amount of surplus water expressed by a water mobility value of from 0.01 to 20 as the connecting substance without using a flocculant aid, a dry binding agent, a flocculating agent, etc.

It is assumed that when the amount of surplus water in the heat generating composition is appropriate, the surplus water causes hydration against hydrophilic groups in the components of the composition due to a bipolar mutual action or hydrogen bond, etc. and that it is present even in the surroundings of hydrophobic groups while having high structural properties. Thus, it is assumed that the heat generating composition becomes in a state of a mud ball, thereby revealing moldability. This is connecting water as a connecting substance in some meaning. Besides, there is water in a state called as free water which can freely move, and it is thought that when the surplus water increases, the structure is softened, whereby the free water increases. Furthermore, controlling factors which an iron powder causes an oxidation reaction are an amount of existing water and a feed amount of oxygen to the surface of the iron powder. It is said that in a degree of water adsorbing film (less than 100 angstroms), the water is not sufficient and that the oxidation rate is small. When the adsorbing film becomes about 1 $\mu$m, the water content becomes sufficient. Furthermore, since the thickness of the water film is thin, feed of oxygen onto the surface of the iron powder becomes easy, whereby the oxidation rate becomes large. It is assumed that when the film becomes thicker to an extent that the adsorbing film exceeds 1 $\mu$m, the feed amount of oxygen is reduced. The present inventors have obtained knowledge that the water mobility value expressing the optimal water content at which moldability and oxidation rate in fixed levels or more are revealed is from 0.01 to 20, leading to accomplishment of the invention.

That is, by using an appropriate amount of surplus water, the respective component particles are coupled with each other by a surface tension of water, moldability is generated in the heat generating composition, and the water does not substantially function as a barrier layer. Thus, the heat generating composition comes into contact with air to generate heat. In addition, by using a heat generating composition using an active iron powder or an active heat generating composition using an active iron powder, the heat generating composition becomes a heat generating composition having remarkably excellent exothermic rising properties and high moldability. Furthermore, heat generation occurs without causing transfer of the water in the heat generating composition molded body as produced by a molding and laminating system into a packaging material or water absorptive sheet. In addition, by providing plural sectional exothermic parts of the heat generating composition molded body as sectioned by seal parts, it is possible to provide a heat generating body which has flexibility itself, is excellent in installation in places where flexibility is required, such as various places of a human body and curved bodies, and is excellent in feeling for use.

Furthermore, in the substrate, the covering material and the heat generating composition molded body, by temporarily adhering at least the covering material and the heat generating composition molded body to each other via a sticky layer and then heat sealing the periphery of the heat generating composition molded body and the surroundings of the heat generating body, certainty of heat seal is improved so that it becomes possible to design to make the production speed of a heat generating body high and make the heat seal width small.

The "moldability" as referred to in the invention exhibits that a laminate of the heat generating composition having a cavity or concave die shape can be formed by force-through molding using a trimming die having a cavity or cast molding using a concave die and after molding including mold release, the molding shape of the heat generating composition molded body is held. When the moldability is revealed, since the shape is held until the heat generating composition molded article is at least covered by a covering material and a seal part is formed between the substrate and the covering material, sealing can be achieved in the periphery of the shape with a desired shape. Also, since so-called "spots" which are a collapsed piece of the heat generating composition are not scattered in the seal part, sealing can be achieved without causing cutting in seal. The presence of the spots causes insufficient sealing.

Next, with respect to the moldability, a measurement device, a measurement method and a judgment method will be described below.

1) Measurement device:

With respect to the measurement device, a stainless steel-made molding die (a plate having a size of 2 mm in thickness $\times$ 200 mm in length $\times$ 200 mm in width and having a cavity as treated by R5 in four corners of 60 mm in length x 40 mm in width in a central part thereof) and a fixable leveling plate are disposed above a travelable endless belt, and magnets (two magnets having a size of 12.5 mm in thickness x 24 mm in length $\times$ 24 mm in width are disposed in parallel) are disposed under the endless belt. The magnets should cover a region of the leveling plate and the vicinity thereof and a region larger than a region covered by a cut side (40 mm) vertical to the advancing direction of the cavity of the molding die.

2) Measurement method:

With respect to the measurement method, a stainless steel plate having a size of 1 mm in thickness $\times$ 200 mm in length $\times$ 200 mm in width is placed on the endless belt of the measurement device, a polyethylene film having a size of 70 $\mu$m in thickness $\times$ 200 mm in length $\times$ 200 mm in width is placed thereon, and a stainless steel-made molding die is further placed thereon. Thereafter, a leveling plate is fixed in a position of the cavity of the molding die of 50 mm far from the end portion in the advancing direction of the endless belt, 50 g of a heat generating composition is then placed in the vicinity of the leveling plate between the leveling plate and the cavity, and the heat generating composition is filled in the cavity of the molding die while leveling it by moving the endless belt at 1.8 m/min.

After the molding die has completely passed through the leveling plate, the traveling of the endless belt is stopped. Next, the molding die is removed, and a heat generating composition molded body as laminated on the polyethylene film is observed.

3) Judgment method:

With respect to the judgment method, in the surroundings of the heat generating composition molded body, in the case where any collapsed piece of the heat generating composition molded body exceeding a maximum length of 800 $\mu$m is not present and the number of collapsed pieces of the heat generating composition molded body having a maximum length of from 300 to 800 $\mu$m is not more than 5, it is to be noted that the heat generating composition has moldability. The moldability is an essential property for a heat generating composition to be used in the molding system. If the heat generating composition does not have moldability, it is impossible to produce a heat generating body by the molding system.

The heat generating composition of the invention has resistance to compression. The "resistance to compression" as referred to herein means that a heat generating composition compressed body obtained by compressing a heat generating composition molded body as accommodated in a molding die within the die to such an extent that the thickness is 70 % of the die thickness holds 80 % or more of exothermic rising properties of the exothermic rising properties of the heat generating composition molded body before compression (a difference in temperature between one minute and 3 minutes after starting a heat generation test of the heat generating composition).

Here, the measurement method of exothermic rinsing properties for the resistance to compression will be described below.

1. Heat generating composition molded body:

1) A magnet is provided in the vicinity of a central part of the back side of a polyvinyl chloride-made supporting plate (5 mm in thickness $\times$ 600 mm in length $\times$ 600 mm in width) of a footed supporting table so as to cover a cavity shape of a molding die.

2) A temperature sensor is placed on the central part the surface of the supporting plate.

3) A polyethylene film (25 $\mu$m in thickness × 250 mm in length × 200 mm in width) as provided with an adhesive layer having a thickness of about 80 $\mu$m is stuck onto the supporting plate via a sticky layer such that the center of the polyethylene film is positioned at the sensor.

4) On an underlay plate (280 mm in length × 150 mm in width × 50 $\mu$m to 2 mm in thickness), a polyethylene film (230 mm in length × 155 mm in width × 25 $\mu$m to 100 $\mu$m in thickness) is placed such that one end of the polyethylene film is projected by about 20 mm outside the underlay plate and that one end thereof in the length direction is substantially coincident with one end of the underlay plate.

5) A template (230 mm in length × 120 mm in width × 3 mm in thickness) having a cavity (80 mm in length × 50 mm in width × 3 mm in height) is placed on the polyethylen film placed on the underlay plate; a template is placed on the polyethylene film such that one end thereof in the length direction is fitted to one end where the underlay plate and the polyethylene film are coincident with each other and that in the width direction, one end part of the width of the template is placed at a position of the central part by about 20 mm far from an opposing end to the side where the polyethylene film is projected outward from the underlay plate. Next, the resulting assembly is placed on the supporting plate together with the underlay plate.

6) A sample is placed in the vicinity of the cavity; a force-in die plate is moved along the molding die; the sample is charged into the cavity while stuffing; and the sample is leveled while stuffing along the template plane (force-in die molding), thereby filling the sample in the die.

7) Next, the magnet beneath the supporting plate is removed; the end portion of the projected polyethylene film is pressed; the underlay plate is removed; and the temperature measurement is started.

2. Heat generating composition compressed body:

1) to 6) are the same as in the case of the heat generating composition molded body.

8) A die having a convex having a thickness of 0.9 mm which can substantially tightly come into the cavity in relation of the cavity with an unevenness is fitted to the cavity and compressed by a roll press or plate press to prepare a heat generating composition compressed body having a thickness of 2.1 mm (compressed to 70 % of the die thickness) within the die.

9) The resulting assembly is placed on the supporting plate together with the underlay plate; the magnet beneath the supporting plate is removed; the end portion of the projected polyethylene film is pressed; the underlay plate is removed; and the temperature measurement is started.

With respect to the measurement of the exothermic temperature, the temperature is measured for 5 minutes at a measurement timing of 2 seconds using a data collector, and resistance to compression is judged in terms of a difference in temperature between after elapsing one minute and after elapsing 3 minutes.

The thickness after compression is preferably from 50 to 99.5 %, more preferably from 60 to 99.5 %, and further preferably from 60 to 95 % of the die thickness.

Incidentally, in the invention, it is to be noted that the heat generating composition molded body includes a heat generating composition compressed body.

The invention will be specifically described below with reference to the Examples, but it should not be construed that the invention is limited thereto.

[Brief Description of the Drawings]

[Fig. 1] is a plan view of an embodiment of the microheater of the invention.
[Fig. 2] is a cross-sectional view along the line Z-Z of the same.
[Fig. 3] is a cross-sectional view of another embodiment of the microheater of the invention.
[Fig. 4] is a plan view of another embodiment of the microheater of the invention.
[Fig. 5] is a cross-sectional view of a still another embodiment of the microheater of the invention.
[Fig. 6] is a plan view of a filter paper for the measurement of water mobility value in the invention.

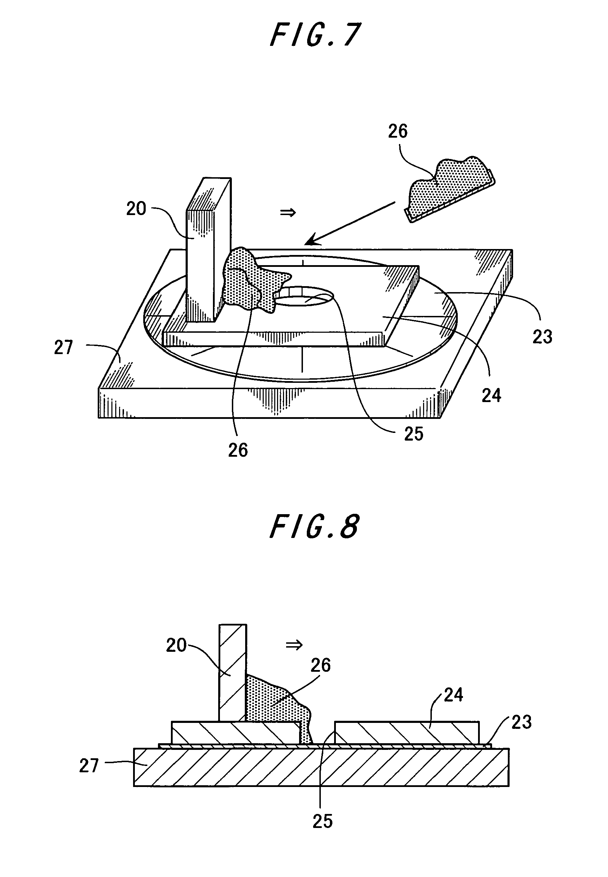

[Fig. 7] is an oblique view for explaining the measurement of water mobility value in the invention.
[Fig. 8] is a cross-sectional view for explaining the measurement of water mobility value in the invention.
[Fig. 9] is a cross-sectional view of the same.
[Fig. 10] is a plan view of a filter paper after carrying out the measurement of water mobility value in the invention.

[Description of Reference Numerals and Signs]

| | |
|---|---|
| 1: | Microheater |
| 2: | Exothermic part |
| 2B: | Heat generating composition molded body |
| 3: | Substrate |
| 4: | Covering material |
| 5: | Netlike hot melt based adhesive layer |
| 6: | Seal part |
| 7: | Adhesive layer |
| 7a: | Separator |
| 20: | Pushing plate |
| 22: | Flat plate |
| 22A: | Non-absorptive film |
| 23: | Filter paper in which eight lines are drawn radiating from the central point with an interval of 45° |
| 24: | Die plate |
| 25: | Hole |
| 26: | Sample |
| 27: | Stainless steel plate |
| 28: | Distance to the oozed-out locus of water or aqueous solution |
| 29: | Position corresponding to a hollow cylindrical hole on filter paper |

[Examples]

(Example 1)

A moldable heat generating composition having a water mobility value of 10, which is a mixture of 100 parts by weight of a reduced iron powder (particle size: not more than 300 μm), 5.0 parts by weight of active carbon and 3 % of salt water, was used.

Next, the heat generating composition was laminated on the surface of a polyethylene film of a substrate 3 made of a separator-provided polyethylene film provided with an SIS based adhesive layer having a thickness of 30 μm by force-through molding using a trimming die provided with a cavity of 8 mm in width × 50 mm in length × 3 mm in thickness, thereby forming a heat generating composition molded body 2 constituting an exothermic part 2B; next, an air-permeable covering material 4 having a nylon-made non-woven fabric with a basis weight of 40 g/m² laminated on a perforated polyethylene film was covered thereon such that the polyethylene film side was faced at the heat generating composition molded body 2B; and the surroundings of the respective heat generating composition molded body 2B was sealed in a seal width of 8 mm, thereby obtaining an adhesive layer-provided microheater 1 having an external dimension of 26 mm in width × 68 mm in length (see Figs. 1 and 2).

Incidentally, the air permeability of the air-permeable covering material 4 was 1, 000 g/m²/24 hr in terms of a moisture permeability by the Lyssy method.

Collapsed pieces of the heat generating composition molded body were not observed in the surroundings of the heat generating composition molded body, heat sealing could be surely achieved, and cutting in seal was not caused.

This microheater was sealed and accommodated in an outer bag and allowed to stand at room temperature for 24 hours. After 24 hours, the microheater was taken out from the outer bag and then subjected to an exothermic test. As a result, the temperature reached 42 °C after 7 minutes and became 35 °C after 3 hours. This microheater was subjected to an exothermic test of the body by sticking onto an acupuncture point of the shoulder. As a result, it exhibited a sufficient moxibustion effect and excellent usefulness.

(Comparative Example 1)

A heat generating composition was produced in the same manner as in Example 1, except for changing the water mobility value to not more than 0. A microheater was produced in the same manner as in Example 1. However, collapsed pieces of the heat generating composition laminate were scattered in the periphery of the heat generating composition molded body, and cutting in seal was caused in the seal part.

This microheater was subjected to an exothermic test of the body. As a result, the temperature excessively raised and the temperature was not uniform so that it could not be used as a microheater.

(Example 2)

A microheater having an external dimension of 31 mm in diameter in a seal width of 3 mm was obtained in the same manner as in Example 1, except for using a packaging material having a non-woven fabric laminated on a perforated polyethylene film as a substrate and a covering material, respectively.

The air permeability of the substrate and the covering material was 1,000 g/m$^2$/24 hr, respectively in terms of a moisture permeability by the Lyssy method.

Next, a separator-provided pressure sensitive adhesive double coated tape of 1 mm in thickness × 24 mm in diameter was stuck on one surface of the microheater. Next, the microheater was accommodated in an outer bag and allowed to stand at room temperature for 24 hours. After 24 hours, the microheater was taken out from the outer bag and then subjected to an exothermic test. As a result, the temperature of the side at which the pressure sensitive adhesive double coated tape was not provided reached 41 °C after 6 minutes, and after keeping substantially 42 °C, the temperature became 35 °C after 3 hours. This microheater was subjected to an exothermic test of the body by sticking onto an acupuncture point of the shoulder. As a result, it exhibited a sufficient moxibustion effect and excellent usefulness.

(Example 3)

By using the exothermic part of Example 1, a microheater using a pedestal in place of the adhesive layer was prepared (see Fig. 4). The pedestal is constituted by providing an adhesive layer 7 on the both surfaces of a backing made of a polyethylene expanded body. Incidentally, a separator is provided on the adhesive layer 7 in the sticking side to a human body or the like. Incidentally, an embodiment as shown in Fig. 5 is concerned with one which the air-permeable covering material 4 is contact bonded to the heat generating composition molded body 2B.

(Example 4)

A microheater was prepared by changing the shape of the microheater of Example 1 to a triangular shape (see Fig. 4).

(Example 5)

A reaction mixture consisting of 100 parts by weight of a reduced iron powder (particle size: not more than 300 μm), 5 parts by weight of active carbon and 5 parts by weight of 11 % salt water and having a water mobility value of less than 0.01 was charged in a contact treatment device vessel.

Next, the upper portion of the contact treatment device vessel was opened to air, and the reaction mixture was stirred in the opened state to air under circumferences at 20 °C. At a point of time when the temperature rise of the reaction mixture reached 15 °C, the reaction mixture was sealed in an air-impermeable accommodating bag, thereby obtaining a heat generating mixture.

Next, as shown in Fig. 5, the moldable heat generating composition was laminated on the surface of a polyethylene film of a substrate 3 made of a separator-provided polyethylene film provided with an SIS based adhesive layer having a thickness of 30 μm by force-through molding using a trimming die provided with a cavity of 8 mm in diameter × 3 mm in height, thereby forming a heat generating composition molded body 2 constituting an exothermic part 2B; next, an air-permeable covering material 4 having a nylon-made non-woven fabric with a basis weight of 40 g/m$^2$ laminated on a perforated polyethylene film was covered thereon such that the polyethylene film side was faced at the heat generating composition molded body 2B; and the surroundings of the respective heat generating composition molded body 2B was sealed in a seal width of 8 mm, thereby obtaining an adhesive layer-provided microheater 1 having the same external dimension as the shape in Example 1 and having a diameter of 28 mm.

Incidentally, the air permeability of the air-permeable covering material 4 was 1,000 g/m$^2$/24 hr in terms of a moisture permeability by the Lyssy method.

Collapsed pieces of the heat generating composition molded body were not observed in the surroundings of the heat generating composition molded body, heat sealing could be surely achieved, and cutting in seal was not caused.

This microheater was accommodated in an outer bag and allowed to stand at room temperature for 24 hours. After 24 hours, the microheater was taken out from the outer bag and then subjected to an exothermic test. As a result, the temperature reached 40 °C after 5 minutes, and substantially 42 °C was kept for about 3 hours. This microheater was subjected to an exothermic test of the body by sticking onto an acupuncture point of the shoulder. As a result, it exhibited a sufficient moxibustion effect and excellent usefulness.

(Comparative Example 2)

A microheater was produced in the same manner as in Example 6, except that the oxidizing gas contact treatment was not carried out. This microheater was sealed and accommodated in an air-impermeable accommodating bag and allowed to stand at room temperature for 24 hours. After 24 hours, the microheater was taken out from the outer bag and then subjected to an exothermic test. As a result, it took 5 minutes to reach 38 °C.

(Example 6)

A moldable heat generating composition having a water mobility value of 8, which is a mixture of 100 parts by weight of a reduced iron powder (particle size: not more than 300 $\mu$m), 3.0 parts by weight of a water retaining agent (particle size: not more than 300 $\mu$m), 5.0 parts by weight of active carbon (particle size: not more than 300 $\mu$m) and 11 % of salt water, was used.

Next, as shown in Fig. 5, by using the heat generating composition, a heat generating composition molded body 2B (8 mm in width $\times$ 100 mm in length $\times$ 3 mm in height) was provided on one surface of a substrate 3 made of a polyethylene film by force-through molding using a trimming die provided with a cavity of 8 mm in width $\times$ 100 mm in length $\times$ 3 mm in thickness; next, an air-permeable covering material 4 having a nylon-made non-woven fabric with a basis weight of 40 g/m$^2$ laminated on a polyethylene-made porous film was covered thereon such that the porous film surface was brought into contact with the heat generating composition molded body 2B; and the surroundings of the respective heat generating composition molded body 2B was sealed in a seal width of 8 mm, thereby obtaining a microheater 1 having an external dimension of 28 mm in width x 116 mm in length (see Fig. 4).

Incidentally, the air permeability of the air-permeable covering material 4 was 400 g/m$^2$/24 hr in terms of a moisture permeability by the Lyssy method. Furthermore, both the substrate and the covering material had a stretching ratio of less than 1.2. Collapsed pieces were not observed in the surroundings of the heat generating composition molded body, and heat sealing could be surely achieved.

This microheater was accommodated in an outer bag and allowed to stand at room temperature for 24 hours. After 24 hours, the microheater was taken out from the outer bag and then subjected to an exothermic test. As a result, the temperature reached 38 °C within 3 minutes. Since the microheater of this Example has a rectangular shape, it can be struck along a muscle or a tendon of an affected part and therefore, it is effective for relieving various symptoms.

(Example 7)

A batchwise stirring tank composed of a mixer equipped with a rotary blade in a blade form of a ventilation fan was used as an oxidizing gas contact treatment device, and air was used as an oxidizing gas.

First of all, a reaction mixture consisting of 100 parts by weight of a reduced iron powder (particle size: not more than 300 $\mu$m), 5.3 parts by weight of active carbon (particle size: not more than 300 $\mu$m), 3.5 parts by weight of a wood meal (particle size: not more than 300 $\mu$m), 3.0 parts by weight of a water absorptive polymer (particle size: not more than 300 $\mu$m), 0.2 parts by weight of calcium hydroxide, 0.7 parts by weight of sodium sulfite and 5 parts by weight of 11 % salt water and having a water mobility value of less than 0.01 was charged in the contact treatment device vessel.

Next, the upper portion of the contact treatment device vessel was opened to air, the reaction mixture was subjected to self heat generation with stirring in the opened state to air under circumstances at 20 °C at a maximum temperature of the heat generating composition of 55 °C for 2 minutes, thereby achieving an oxidizing gas contact treatment. This reaction mixture had a wustite content of 10 %. Next, 11 % salt water was added to the heat generating mixture to adjust the water content, thereby obtaining a moldable heat generating composition having a water mobility value of 10.

Next, as shown in Fig. 5, by using the moldable heat generating composition, a heat generating composition molded body 2B constituting an exothermic part 2B was provided on a substrate 3 made of a polyethylene film by force-through molding using a trimming die provided with a cavity of 8 mm in diameter $\times$ 3 mm in height; next, an air-permeable covering material 4 having a nylon-made non-woven fabric with a basis weight of 40 g/m$^2$ laminated on a polyethylene-made porous film was covered thereon such that the porous film surface was brought into contact with the heat generating composition molded body 2B; and the surroundings of the respective heat generating composition molded body 2B was sealed in a seal width of 8 mm, thereby obtaining a microheater 1 of 28 mm in width $\times$ 116 mm in length.

Incidentally, the air permeability of the air-permeable covering material 4 was 400 g/m$^2$/24 hr in terms of a moisture permeability by the Lyssy method. Furthermore, both the substrate and the covering material had a stretching ratio of less than 1.2. Collapsed pieces were not observed in the surroundings of the heat generating composition molded body, and heat sealing could be surely achieved.

This microheater 1 was sealed and accommodated in an outer bag and allowed to stand at room temperature for 24 hours. After 24 hours, the microheater was taken out from the outer bag and then subjected to an exothermic test. As a result, the temperature reached 38 °C within 3 minutes. This microheater 1 can also be incorporated into, for example, a packaging material for fixing the body.

(Example 8)

A heat generating mixture consisting of 100 parts by weight of an iron powder having a wustite content of less than 1 % (particle size: not more than 300 $\mu$m), 25 parts by weight of active carbon (particle size: not more than 300 $\mu$m), 3 parts by weight of a water absorptive polymer (particle size: not more than 300 $\mu$m), 0.5 parts by weight of calcium hydroxide, 0.7 parts by weight of sodium sulfite and 5 parts by weight of 11 % salt water and having a water mobility value of less than 0.01 was charged in a contact treatment device vessel in the same manner as in Example 3.

Next, the upper portion of the contact treatment device vessel was opened to air, and the reaction mixture was subjected to a self heat generation with stirring in the opened state to air under circumferences at 20 °C. At a point of time when the temperature rise of the reaction mixture reached 35 °C, the reaction mixture was accommodated in an air-impermeable accommodating bag, thereby obtaining a heat generating mixture. 11 % salt water was mixed in the heat generating mixture to obtain a moldable heat generating composition having a water mobility value of 9.

Next, as shown in Fig. 5, on the surface of a substrate 3 made of a polyethylene film and provided with an adhesive layer 7 provided with a separator 7a, a heat generating composition molded body 2 constituting an exothermic part 2B was laminated on the polyethylene film at which the sticky layer was not provided by force-through molding using a trimming die provided with a cavity of 10 mm in diameter $\times$ 3 mm in height; next, an SIS based hot melt based adhesive was provided in a netlike form on a porous film of an air-permeable covering material 4 having a nylon-made non-woven fabric with a basis weight of 40 g/m$^2$ laminated on a polyethylene-made porous film by a melt blow method; and after covering such that the netlike adhesive layer was brought into contact with the heat generating composition molded body, the product was contact bonded by a sponge roll, thereby temporarily adhering the heat generating composition molded body and the surroundings thereof.

Thereafter, the surroundings of the respective heat generating composition molded body 2B was sealed in a seal width of 8 mm, thereby obtaining a microheater 1 having an external dimension of 30 mm in diameter.

Incidentally, the air permeability of the air-permeable covering material 4 was 400 g/m$^2$/24 hr in terms of a moisture permeability by the Lyssy method. Furthermore, both the substrate and the covering material had a stretching ratio of less than 1.2. Collapsed pieces of the heat generating composition molded body were not observed in the surroundings of the heat generating composition molded body, heat sealing could be surely achieved, and cutting in seal was not caused. This microheater 1 was accommodated in an outer bag and allowed to stand at room temperature for 24 hours. After 24 hours, the microheater was taken out from the outer bag and then subjected to an exothermic test. As a result, the temperature reached 38 °C within 3 minutes.

This microheater 1 can also be incorporated into, for example, a packaging material for fixing the body.

(Example 9)

An air-impermeable covering material made of an LDPE-covered polypropylene non-woven fabric was bored by 26 pins having a diameter of about 0.5 mm, thereby preparing an air-permeable covering material. By this boring method, the air permeability of the air-permeable covering material was 400 g/m$^2$/24 hr in terms of a moisture permeability by the Lyssy method.

A microheater 1 having a diameter of 30 mm and having the same shape as in Example 1 was prepared in the same manner as in Example 3, except for using the foregoing covering material. This microheater 1 was accommodated in an outer bag and allowed to stand at room temperature for 24 hours. After 24 hours, the microheater 1 was taken out from the outer bag and then subjected to an exothermic test. As a result, the temperature reached 38 °C within 3 minutes.

This microheater 1 can also be incorporated into, for example, a packaging material for fixing the body.

(Example 10)

Next, by using the moldable heat generating composition of Example 2 and using a substrate 3 made of a laminate of a nylon-made non-woven fabric and a polyethylene film and provided with an SIS based adhesive layer having a thickness of 30 $\mu$m and provided with a separator on the side of the nylon-made non-woven fabric, a heat generating composition laminate 2 constituting an exothermic part 2B was laminated on the surface of the polyethylene film by force-through molding using a trimming die provided with a cavity of 8 mm in diameter $\times$ 3 mm in height; next, an air-permeable covering material 4 having a nylon-made non-woven fabric with a basis weight of 40 g/m$^2$ laminated on a polyethylene-made porous film was covered thereon such that the porous film side was brought into contact with the heat generating composition molded body 2B; and the surroundings of the respective heat generating composition molded body 2B was sealed in a seal width of 8 mm, thereby obtaining a sticky layer-provided microheater 1 having an external dimension of 28 mm in diameter.

Incidentally, the air permeability of the air-permeable covering material 4 was 400 g/m$^2$/24 hr in terms of a moisture permeability by the Lyssy method. Furthermore, both the substrate and the covering material had a stretching ratio of less than 1.2, and the covering material and the substrate did not have stretchability. Collapsed pieces were not observed in the surroundings of the heat generating composition molded body, heat sealing could be surely achieved, and cutting in seal was not caused.

Furthermore, the adhesive layer-provided substrate was prepared in the following manner.

5.0 parts by weight of a butyl rubber, 5.0 parts by weight of polyisobutylene, 5.0 parts by weight of a polystyrene butadiene rubber, 25.0 parts by weight of a styrene/isoprene/styrene block copolymer, 15.0 parts by weight of liquid paraffin, and 0.6 parts by weight of silicon dioxide were mixed upon heating under pressure, to which were then added 42.0 of a rosin ester resin, 1.4 parts by weight of a highly water absorptive polymer and 1.0 part by weight of tocopherol acetate to prepare an adhesive.

This microheater 1 was accommodated in an outer bag and allowed to stand at room temperature for 24 hours. After 24 hours, the microheater 1 was taken out from the outer bag and then subjected to an exothermic test of the body by sticking onto an acupuncture point of the shoulder. As a result, it was felt warm within 3 minutes, and the warmth was continued for 3 hours. At the same time, usefulness was evaluated. As a result, all were excellent.

Furthermore, for fifteen subjects who had muscular fatigue of shoulders, the microheater 1 having a drug-incorporated adhesive layer was stuck onto an affected part, and a degree of improvement in the symptom after lapsing 3 hours was evaluated. With respect to the warmth feeling, eleven subjects felt that it was very strong, and four subjects felt that it was strong. With respect to the improvement of the symptom in the affected part, nine subjects felt that a remarkable improvement was obtained, and six subjects felt that a medium improvement was obtained.

Furthermore, all of the subjects appealed that not only this thermal sticking agent did not cause peeling during the use, but also adhesion to an outer cover was good, an uncomfortable feeling was not brought, and a feeling for use was good.

(Example 11)

97.9 % by weight of an adhesive consisting of 91 % by weight of an ethylhexyl acrylate-dodecyl methacrylate copolymer resin and 9 % by weight of isopropyl myristate was dissolved in ethyl acetate, and 2.0 % by weight of vitamin E and 0.1 % by weight of benzyl nicotinate were mixed therewith to obtain an adhesive. A specimen in a rectangular parallelepiped form was prepared in the same manner as in Example 1, and an adhesive layer was provided in the substrate side, thereby obtaining a drug-incorporated adhesive layer-provided microheater 1 of 28 mm in width × 116 mm in length.

For fifteen subjects who had fatigue in calves, the specimen was stuck onto an affected part, and a degree of improvement in the symptom after lapsing 3 hours was evaluated. With respect to the warmth feeling, eleven subjects felt that it was very strong, and four subjects felt that it was strong.

With respect to the improvement of the symptom in the affected part, nine subjects felt that a remarkable improvement was obtained, and six subjects felt that a medium improvement was obtained.

Furthermore, all of the subjects appealed that not only this thermal sticking agent did not cause peeling during the use, but also adhesion to an outer cover was good, an uncomfortable feeling was not brought, and a feeling for use was good.

**Claims**

1. A microheater having a heat generating composition molded body made of a moldable heat generating composition containing surplus water as a connecting substance accommodated in an air-permeable accommodating bag, **characterized in that**:

   1) the accommodating bag is made of a heat seal layer-containing substrate and a covering material and has an exothermic part as formed by laminating a heat generating composition molded body as molded on the substrate which is substantially planar and does not have a pocket, an accommodating division and an accommodating section, covering by the covering material and heat sealing the periphery of the heat generating composition molded body,
   2) the moldable heat generating composition contains, as essential components, an iron powder, a carbon component, a reaction accelerator and water, has a content of water in the moldable heat generating composition of from 1 to 60 %, does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air,

3) a volume of the heat generating composition molded body is from 0.1 to 30 $cm^3$, and the capacity of the exothermic part to a ratio of the volume of the heat generating composition molded body is from 0.6 to 1.0, and

4) a maximum height of the exothermic part is from 0.1 to 10 mm.

2. The microheater according to claim 1, **characterized in that** the shape of the heat generating composition molded body, the exothermic part and the microheater is at least one shape selected from the group consisting of a circular shape, a triangular shape, a star shape, a rectangular shape, a square shape, a flower shape, an elliptical shape, a cubic shape, a parallelepiped shape, a polygonal pyramidal shape, a conical shape, a pillar shape, an elliptic cylindrical shape, semi-pillar shape, a semi-elliptic cylindrical shape, a cylindrical shape, and a spherical shape.

3. The microheater according to claim 2, **characterized in that** the shape of the exothermic part is a pillar shape and has a diameter of from 1 to 50 mm and a maximum height of from 0.1 to 10 mm.

4. The microheater according to claim 2, **characterized in that** the shape of the exothermic part is a parallelepiped shape and has a maximum length of from 5 to 200 mm, a maximum width of from 1 to 50 mm and a maximum height of from 0.1 to 10 mm, and the exothermic part is formed by heat sealing the periphery of the heat generating composition molded body.

5. The microheater according to claim 2, **characterized in that** the shape of the exothermic part is an elliptic cylindrical shape and has a maximum width of from 3 to 30 mm.

6. The microheater according to claim 2, **characterized in that** the exothermic part has a maximum width of from 1 to 50 mm, a maximum height of from 0.1 to 10 mm and a longest length of from 5 to 200 mm.

7. The microheater according to claim 2, **characterized in that** the shape of the exothermic part is a cubic shape and has a maximum width of from 5 to 30 mm, and the exothermic part is formed by heat sealing the periphery of the heat generating composition molded body.

8. The microheater according to claim 1, **characterized in that** at least the heat generating composition molded body is compressed.

9. The microheater according to claim 1, **characterized in that** the heat seal part is formed by heat sealing after temporary adhesion by an adhesive layer as formed on the heat seal layer, and an adhesive component which constitutes the adhesive layer and a heat seal material component which constitutes the heat seal layer are copresent in the heat seal part.

10. The microheater according to claim 1, **characterized in that** the moldable heat generating composition contains a component resulting from a contact treatment of a mixture containing at least an iron powder, a carbon component, a reaction accelerator and water as essential components with an oxidizing gas.

11. The microheater according to claim 1, **characterized in that** the iron powder comprising particles, a surface of each of which is at least partially covered with an iron oxide film, the oxide film has a thickness of 3 nm or more, and the iron powder at least contains from 20 to 100 % by weight of an active iron powder particle having a region of an oxygen-free iron component in at least one region selected from a central part region of the iron powder particle and a region beneath the iron oxide film.

12. The microheater according to claim 1, **characterized in that** the iron powder comprising particles, a surface of each of which is at least partially covered with a wustite film and contains from 20 to 100 % by weight of an active iron powder having an amount of wustite of from 2 to 50 % by weight in terms of an X-ray peak intensity ratio to iron.

13. The microheater according to claim 1, **characterized in that** the moldable heat generating composition contains at least one member selected from additional components consisting of a water retaining agent, a water absorptive polymer, a pH adjusting agent, a hydrogen formation inhibitor, an aggregate, a fibrous material, a functional substance, a surfactant, an organosilicon compound, a pyroelectric substance, a moisturizer, a fertilizer component, a hydrophobic polymer compound, a heat generating aid, a metal other than iron, a metal oxide other than iron oxide, an acidic substance, and a mixture thereof.

**14.** The microheater according to claim 1, **characterized in that** 80 % or more of a non-water soluble solid component which constitutes the moldable heat generating composition has a particle size of not more than 300 $\mu$m and a maximum particle size of not more than 1 mm.

**15.** The microheater according to claim 1, **characterized in that** in the substrate or the covering material, a sticky layer is laminated as a fixing measure on at least a part of the exposed surface thereof.

**16.** A process for producing a microheater having a heat generating composition molded body accommodated in an air-permeable accommodating bag, **characterized in that**:

1) a moldable heat generating composition containing surplus water as a connecting substance is molded, the heat generating composition molded body is laminated on a substrate which is substantially planar and does not have an accommodating pocket, the heat generating composition molded body is covered by a covering material, and the periphery of the heat generating composition molded body is heat sealed to form an exothermic part,
2) the moldable heat generating composition contains, as essential components, an iron powder, a carbon component, a reaction accelerator and water, has a content of water in the moldable heat generating composition of from 1 to 60 %, does not contain a flocculant aid, a flocculant, an agglomeration aid, a dry binder, a dry binding agent, a dry binding material, a sticky raw material, a thickener and an excipient, contains surplus water so as to have a water mobility value of from 0.01 to 20, with the water in the heat generating composition not functioning as a barrier layer, and is capable of causing an exothermic reaction upon contact with air,
3) a volume of the heat generating composition molded body is from 0.1 to 30 cm$^3$, and a ratio of the capacity of the exothermic part to the volume of the heat generating composition molded body is from 0.6 to 1.0, and
4) a maximum height of the exothermic part is from 0.1 to 10 mm.

**17.** The process for production a microheater according to claim 16, **characterized in that** at least the periphery of the heat generating composition molded body is heat sealed after temporary adhesion of the substrate and the covering material via a sticky layer.

*FIG.1*

*FIG.2*

*FIG.3*

*FIG. 4*

*FIG.5*

*FIG.6*

## FIG.7

## FIG.8

# FIG.9

# FIG.10

<table>
<tr><td colspan="2" align="center"><b>INTERNATIONAL SEARCH REPORT</b></td><td>International application No.<br>PCT/JP2005/013006</td></tr>
</table>

| | |
|---|---|
| **A. CLASSIFICATION OF SUBJECT MATTER**<br>Int.Cl$^7$ A61H39/06, A61F7/08 | |

According to International Patent Classification (IPC) or to both national classification and IPC

**B. FIELDS SEARCHED**

Minimum documentation searched (classification system followed by classification symbols)
Int.Cl$^7$ A61H39/06, A61F7/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2003-334212 A (Maikoru Kabushiki Kaisha),<br>25 November, 2003 (25.11.03),<br>Full text; all drawings<br>(Family: none) | 1,9,10,13-17<br>2-8,12 |
| Y | JP 2003-509120 A (The Procter & Gamble Co.),<br>11 March, 2003 (11.03.03),<br>Claims 4, 9, 10<br>& EP 1212020 A       & WO 2001/019302 A1 | 2-7 |
| Y | JP 2002-155273 A (Kaoru USUI),<br>28 May, 2002 (28.05.02),<br>Claim 9<br>(Family: none) | 8 |

| | | | |
|---|---|---|---|
| ☒ Further documents are listed in the continuation of Box C. | | ☐ See patent family annex. | |

| | |
|---|---|
| *    Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| | |
|---|---|
| Date of the actual completion of the international search<br>27 September, 2005 (27.09.05) | Date of mailing of the international search report<br>25 October, 2005 (25.10.05) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

| International application No. |
| --- |
| PCT/JP2005/013006 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 10-17907 A  (Dowa Iron Powder Co., Ltd.),<br>20 January, 1998 (20.01.98),<br>Par. Nos. [0005] to [0011]<br>(Family: none) | 12 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 7136233 A
- JP 2000254205 A
- JP 4293989 A
- JP 6343658 A
- JP 7194641 A
- JP 59189183 A
- WO 0013626 A
- JP 9075388 A
- JP 60101448 A
- JP 9276317 A
- JP 11299817 A
- JP 11508314 T
- JP 6026829 A
- JP 2000288008 A
- JP 11508786 T
- JP 2002514104 T
- JP 2002200108 A
- JP 10265373 A
- JP 9087173 A
- JP 6145050 A
- JP 6199660 A
- JP 10279466 A
- JP 10182408 A
- JP 3161605 B
- JP 2001507593 T